(19) Europäisches Patentamt
European Patent Office
Office européen des brevets

(11) EP 2 003 129 B1

(12) EUROPEAN PATENT SPECIFICATION

(45) Date of publication and mention of the grant of the patent:
**09.08.2017 Bulletin 2017/32**

(51) Int Cl.:
*C07D 471/04* (2006.01) *C07D 401/04* (2006.01)

(21) Application number: **08157576.3**

(22) Date of filing: **04.06.2008**

(54) **1H-Pyrido[3,4-B]indol-1-one and 2,3,4,9-Tetrahydro-1H-Beta-Carbolin-1-one Derivatives**

1H-Pyrido[3,4-B]indol-1-on und 2,3,4,9-Tetrahydro-1H-Beta-Carbolin-1-on-Derivate

Dérivés de 1H-pyrido[3,4-B]indol-1-one et de 2,3,4,9-tétrahydro-1H-bêta-carbolin-1-one

(84) Designated Contracting States:
**DE FR GB IT**

(30) Priority: **11.06.2007 EP 07110007**

(43) Date of publication of application:
**17.12.2008 Bulletin 2008/51**

(73) Proprietor: **Nerviano Medical Sciences S.r.l.**
**20014 Nerviano (MI) (IT)**

(72) Inventors:
- **Cervi, Giovanni**
  **22100, Como (IT)**
- **Felder, Eduard**
  **CH-6850, Mendrisio (CH)**
- **Papeo, Gianluca Mariano Enrico**
  **23870, Cernusco Lombardone (LC) (IT)**
- **Vulpetti, Anna**
  **20047, Brugherio (MI) (IT)**
- **Gennari, Cesare**
  **20133, Milan (IT)**
- **La Ferla, Barbara**
  **20126, Milan (IT)**
- **Nicotra, Francesco**
  **20126, Milan (IT)**
- **Riccaboni, Mauro**
  **20020- Legnano (Milan) (IT)**

(74) Representative: **Mazzini, Giuseppe**
**Nerviano Medical Sciences S.r.l.**
**Patent Department**
**Viale Pasteur, 10**
**20014 Nerviano (Milano) (IT)**

(56) References cited:
**WO-A-01/98299**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 2 003 129 B1

## Description

## BACKGROUND OF THE INVENTION

The malfunctioning of protein kinases (PKs) is the hallmark of numerous diseases. A large share of the oncogenes and proto-oncogenes involved in human cancers code for PKs. The enhanced activities of PKs are also implicated in many non-malignant diseases, such as benign prostate hyperplasia, familial adenomatosis, polyposis, neuro-fibromatosis, psoriasis, vascular smooth cell proliferation associated with atherosclerosis, pulmonary fibrosis, arthritis glomerulonephritis and post-surgical stenosis and restenosis. PKs are also implicated in inflammatory conditions and in the multiplication of viruses and parasites. PKs can also play a major role in the pathogenesis and development of neurodegenerative disorders. PKs malfunctioning and disregulation are further discussed in Current Opinion in Chemical Biology 1999, 3, 459 - 465.

Among the several protein kinases known in the art as being implicated in the growth of cancer cells is Cdc7, an evolutionary conserved serine-threonine kinase which plays a pivotal role in linking cell cycle regulation to genome duplication, being essential for the firing of DNA replication origins (see Montagnoli A. et al., EMBO Journal, Vol. 21, No. 12, pp. 3171-3181, 2002; Montagnoli A. et al., Cancer Research Vol. 64, October 1, pp. 7110-7116, 2004).

WO 03/022849 describes carbazole derivatives and their use as NPY5 receptor antagonists.

In WO00/002878 the use of beta-carboline derivatives is claimed for the treatment or prevention of diseases or disorders by selective action at imidazoline receptors.

Several heterocyclic compounds are known in the art as protein kinase inhibitors. Among them are, for instance, pyrrolo-pyrazoles disclosed in WO 02/12242; tetrahydroindazoles disclosed in WO 00/69846; pyrrolo-pyridines disclosed in WO 01/98299; aminophthalazinones disclosed in WO 03/014090 and aminoindazoles disclosed in WO 03/028720.

In addition, pyrrolopyridinone derivatives for the treatment of obesity are disclosed in the patent WO2003027114 to Bayer Pharmaceuticals Corporation. In particular a pyridylpyrrolopyridinone, namely 5-cyclohexyl-1-(2,4-dichlorophenyl)-3-methyl-2-pyridin-3-yl-1,5,6,7-tetrahydro-pyrrolo[3,2-c]pyridin-4-one is reported. Pyrrolopyridinone derivatives and other heterocyclic compounds, claimed to be endowed with mitogen activated protein kinase-activated protein kinase-2 inhibitory activity, are disclosed in WO2004058762 and US20060276496.

The following document pertains to carboline compounds as inhibitors of mitogen activated proteases:"WU JIANG-PING ET AL: "The discovery of carboline analogs as potent MAPKAP-K2 inhibitors", BIOORGANIC & MEDICINAL CHEMISTRY LETTERS, vol.17, no.16, pages 4664-4669, XP029094751,ISSN: 0960-894X, DOI: 10.1016/J.BMCL.2007.05.101".

Accordingly, there is a need for new compounds that can inhibit protein kinase activity.

## SUMMARY OF THE INVENTION

The invention relates to novel compounds which are useful, in therapy, as agents against a host of diseases caused by and/or associated to a disregulated protein kinase activity and, more particularly, Cdk2 or Cdc7 activity.

In another aspect, the inventive compounds are also useful as aurora 1 or aurora 2 inhibitors.

The invention also relates to compounds which have protein kinase inhibiting activity and, more particularly, Cdk2 or Cdc7 inhibiting activity.

One aspect of the invention relates to 1H-pyrido[3,4-b]indol-1-one and 2,3,4,9-tetrahydro-1H-beta-carbolin-1-one derivatives which are represented by formula (I)

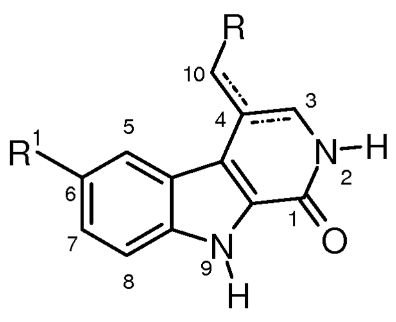

**(I)**

wherein

the lines between carbon 3 and carbon 4; and between carbon 4 and carbon 10, represent a single or double bond with the provisos that the bonds between C3-C4 and C4-C10 cannot be both double bonds;
**R** is selected from the group consisting of $CH_2OH$, $-CH_2OCOR^2$, $-CONR^2R^3$, and $-COOR^2$;
**$R^1$** is selected from the group consisting of $-NO_2$, $-NHR^4$, $-NHCOR^4$, $-NHCONR^4R^5$, $-NHSO_2R^4$ and $-NHCOOR^4$; wherein $R^2$, $R^3$, $R^4$, and $R^5$, which can be the same or different, are each independently selected from the group consisting of hydrogen, $(C_1-C_6)$alkyl, $(C_2-C_6)$alkenyl, $(C_2-C_6)$alkynyl, $(C_3-C_6)$cycloalkyl, cycloalkyl$(C_1-C_6)$alkyl, carbocyclic, $(C_1-C_6)$alkyl heterocycle, aryl, aryl$(C_1-C_6)$alkyl, $(C_1-C_6)$alkylaryl, heterocycle, and heterocycle$(C_1-C_6)$alkyl, or
either $R^2$ and $R^3$ as well as $R^4$ and $R^5$, taken together with the nitrogen atom to which they are bonded, can each form an optionally substituted 4 to 7 membered heterocycle, containing one to three heteroatoms or heteroatomic groups selected from S, O, N or NH;
or a pharmaceutically acceptable salt or solvate thereof.

Preferably, R is $-CONR^2R^3$ and $R^1$ is $-NHR^4$ or $-NHCOR^4$.

More preferably, $R^1$ is $-NHCOR^4$ and R is $-CONR^2R^3$ wherein $R^2$ and $R^3$ are each independently hydrogen or alkyl.

Another aspect of the invention relates to a pharmaceutical composition comprising an amount of the compound of Formula (I), or a pharmaceutically acceptable salt or solvate thereof, and a pharmaceutically acceptable carrier.

Another aspect of the invention relates to a method for treating cell proliferative disorders or conditions that can be caused by and/or associated with an altered protein kinase activity, by administering to a mammal in need of said treatment an amount of a N-substituted pyrrolopyridinone derivative represented by formula (I).

Another aspect of the invention relates to a method of treating cell proliferative disorders caused by and/or associated with an altered Cdk2 or Cdc7 kinase activity. Another aspect of the invention relates to a method of antagonizing activity toward Cdk2 or Cdc7, comprising administering to said Cdk2 or Cdc7 an amount of a compound of Formula (I) that is effective in antagonizing activity toward Cdk2 or Cdc7.

Another aspect of the invention relates to a method of treating a disorder or condition in a mammal, wherein antagonist activity toward Cdk2 or Cdc7 is needed in said mammal, comprising administering to said mammal an amount of a compound of Formula (I) that is effective in antagonizing activity toward Cdk2 or Cdc7.

Another aspect of the invention relates to a method of treating a disorder or condition in a mammal for which antagonist activity toward Cdk2 or Cdc7 is needed in said mammal, comprising administering to said mammal an amount of a compound of Formula (I) that is effective in treating said disorder or condition.

Another aspect of the invention relates to a method of antagonizing activity toward aurora 1 or aurora 2, comprising administering to said aurora 1 or aurora 2 an amount of a compound of Formula (I) that is effective in antagonizing activity toward aurora 1 or aurora 2.

Another aspect of the invention relates to a method of treating a disorder or condition in a mammal, wherein antagonist activity toward aurora 1 or aurora 2 is needed in said mammal, comprising administering to said mammal an amount of a compound of Formula (I) that is effective in antagonizing activity toward aurora 1 or aurora 2.

Another aspect of the invention relates to a method of treating a disorder or condition in a mammal for which antagonist activity toward aurora 1 or aurora 2 is needed in said mammal, comprising administering to said mammal an amount of a compound of Formula (I) that is effective in treating said disorder or condition.

Another aspect of the invention relates to a method of treating a disorder or condition selected from the group consisting of squamous cell carcinoma, hematopoietic tumors of myeloid or lymphoid lineage, tumors of mesenchymal origin, tumors of the central and peripheral nervous system, melanoma, seminoma, teratocarcinoma, osteosarcoma, xeroderma pigmentosum, keratoxanthoma, thyroid follicular cancer, and Kaposi's sarcoma, in a mammal, comprising administering to said mammal in need of said treatment an amount of a compound of Formula (I) that is effective in treating said condition or disorder.

Another aspect of the invention relates to a method of treating a disorder or condition selected from the group consisting of benign prostate hyperplasia, familial adenomatosis, polyposis, neuro-fibromatosis, psoriasis, vascular smooth cell proliferation associated with atherosclerosis, pulmonary fibrosis, arthritis glomerulonephritis, post-surgical stenosis and restenosis, in a mammal, comprising administering to said mammal in need of said treatment an amount of a compound of Formula (I) that is effective in treating said condition or disorder.

Another aspect of the invention relates to a method of treating a disorder or condition selected from the group consisting of carcinoma, squamous cell carcinoma, hematopoietic tumors of myeloid or lymphoid lineage, tumors of mesenchymal origin, tumors of the central and peripheral nervous system, melanoma, seminoma, teratocarcinoma, osteosarcoma, xeroderma pigmentosum, keratoxanthoma, thyroid follicular cancer, and Kaposi's sarcoma, in a mammal, comprising administering to said mammal in need of said treatment an amount of a compound of Formula (I) that is effective in antagonizing activity toward Cdk2 or Cdc7.

Another aspect of the invention relates to a method of treating a disorder or condition selected from the group

consisting of benign prostate hyperplasia, familial adenomatosis, polyposis, neuro-fibromatosis, psoriasis, vascular smooth cell proliferation associated with atherosclerosis, pulmonary fibrosis, arthritis glomerulonephritis, post-surgical stenosis and restenosis, in a mammal, comprising administering to said mammal in need of said treatment an amount of a compound of Formula (I) that is effective in antagonizing activity toward Cdk2 or Cdc7.

Another aspect of the invention relates to a method of treating a disorder or condition selected from the group consisting of squamous cell carcinoma, leukemia, acute lymphocitic leukemia, acute lymphoblastic leukemia, B-cell lymphoma, T-celllymphoma, Hodgkin's lymphoma, non-Hodgkin's lymphoma, hairy cell lymphoma and Burkett's lymphoma, acute and chronic myelogenous leukemias, myelodysplastic syndrome, promyelocytic leukemia, fibrosarcoma, rhabdomyosarcoma, astrocytoma, neuroblastoma, glioma, schwannomas, melanoma, seminoma, teratocarcinoma, osteosarcoma, xeroderma pigmentosum, keratoxanthoma, thyroid follicular cancer , Kaposi's sarcoma and carcinoma of the bladder, breast, colon, kidney, liver, lung, esophagus, gall-bladder, ovary, pancreas, stomach, cervix, thyroid, prostate, or skin, in a mammal, comprising administering to said mammal in need of said treatment an amount of a compound of Formula (I) that is effective in treating said condition or disorder.

Another aspect of the invention relates to a method of treating a disorder or condition selected from the group consisting of squamous cell carcinoma, leukemia, acute lymphocitic leukemia, acute lymphoblastic leukemia, B-cell lymphoma, T-cell-lymphoma, Hodgkin's lymphoma, non-Hodgkin's lymphoma, hairy cell lymphoma and Burkett's lymphoma, acute and chronic myelogenous leukemias, myelodysplastic syndrome, promyelocytic leukemia, fibrosarcoma, rhabdomyosarcoma, astrocytoma, neuroblastoma, glioma, schwannomas, melanoma, seminoma, teratocarcinoma, osteosarcoma, xeroderma pigmentosum, keratoxanthoma, thyroid follicular cancer, Kaposi's sarcoma and carcinoma of the bladder, breast, colon, kidney, liver, lung, esophagus, gall-bladder, ovary, pancreas, stomach, cervix, thyroid, prostate, or skin, in a mammal, comprising administering to said mammal in need of said treatment an amount of a compound of Formula (I) that is effective in antagonizing activity toward Cdk2 or Cdc7.

A more complete appreciation of the invention, and many of the attendant advantages thereof, will be readily understood as the same becomes better understood by reference to the following detailed description.

## DETAILED DESCRIPTION OF THE INVENTION

One aspect of the invention relates to 1H-pyrido[3,4-b]indol-1-one and 2,3,4,9-tetrahydro-1H-beta-carbolin-1-one derivatives which are represented by formula (I)

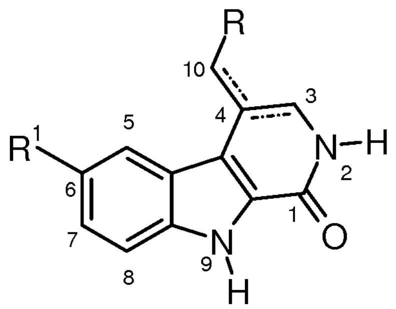

**(I)**

wherein

the lines between carbon 3 and carbon 4; and between carbon 4 and carbon 10, represent a single or double bond with the provisos that the bonds between C3-C4 and C4-C10 cannot be both double bonds;
**R** is selected from the group consisting of $CH_2OH$, $-CH_2OCOR^2$, $-CONR^2R^3$, and $-COOR^2$;
**$R^1$** is selected from the group consisting of $-NO_2$, $-NHR^4$, $-NHCOR^4$, $-NHCONR^4R^5$, $-NHSO_2R^4$ and $-NHCOOR^4$;
wherein $R^2$, $R^3$, $R^4$, and $R^5$, which can be the same or different, are each independently selected from the group consisting of hydrogen $(C_1-C_6)$alkyl, $(C_2-C_6)$alkenyl, $(C_2-C_6)$alkynyl, $(C_3-C_6)$cycloalkyl, cycloalkyl$(C_1-C_6)$alkyl, carbocyclic, $-(C_1-C_6)$alkyl heterocycle, aryl, -aryl$(C_1-C_6)$alkyl, $-(C_1-C_6)$alkylaryl, heterocycle, and heterocycle$(C_1-C_6)$alkyl, or
either $R^2$ and $R^3$ as well as $R^4$ and $R^5$, taken together with the nitrogen atom to which they are bonded, can each form an optionally substituted 4 to 7 membered heterocycle, containing one to three heteroatoms or heteroatomic groups selected from S, O, N or NH; or a pharmaceutically acceptable salt or solvate thereof.

It is to be understood that only one double bond to carbon 4 can exist as single E or Z isomers or a mixture of

them. In addition, when both C3-C4 and C4-C10 are single bonds, the corresponding stereoisomers, either in pure form or in a racemic mixture are intended to be covered by the present invention.

Preferably, R is $-CONR^2R^3$ and $R^1$ is $NHR^4$ or $NHCOR^4$.

More preferably, R is $-CONR^2R^3$ wherein $R^2$ and $R^3$ are each independently hydrogen or alkyl, and $R^1$ is $-NHCOR^4$.

The compounds of formula (I) of the invention can have asymmetric carbon atoms and can therefore exist as individual optical isomers, as racemic admixtures or as any other admixture including a majority of one of the two optical isomers, which are all to be intended as comprised within the scope of the present invention.

Likewise, the use as an antitumor agent of all the possible isomers and their admixtures and of both the metabolites and the pharmaceutically acceptable bio-precursors (otherwise referred to as pro-drugs) of the compounds of formula (I) are also within the scope of the present invention. Prodrugs are any covalently bonded compounds which release the active parent drug, according to formula (I), in vivo.

In cases when compounds can exist in tautomeric forms, for instance keto-enol tautomers, each tautomeric form is contemplated as being included within this invention whether existing in equilibrium or predominantly in one form.

Except where stated otherwise, the following definitions apply throughout the present specification and claims. These definitions apply regardless of whether a term is used by itself or in combination with other terms. Hence the definition of "alkyl" applies to "alkyl" as well as to the "alkyl" portions of "alkylamino", "dialkylamino" etc.

As used above, and throughout the specification, the following terms, unless otherwise indicated, shall be understood to have the following meanings:

"Mammal" means humans and other animals.

"Treating" refers to, and includes, reversing, alleviating, inhibiting the progress of, or preventing, a disease, disorder or condition, or one or more symptoms thereof; and, "treatment" and "therapeutically" refer to the act of treating, as defined above.

The term "effective amount" means an amount of compound of the present invention that is capable of treating a specific disease or antagonizing a specific enzyme, such as a specific protein kinase. The particular dose of compound administered according to the invention will be determined by the particular circumstances surrounding the case including, for example, the compound administered, the route of administration, the state of being of the subject, and the severity of the pathological condition being treated.

"Alkyl" means an aliphatic hydrocarbon group, which can be straight or branched.

Branched means that one or more lower alkyl groups such as methyl, ethyl or propyl, are attached to a linear alkyl chain. $(C_1-C_6)$alkyl means an alkyl group that is 1-6 carbon atoms long. The alkyl group can be substituted by one or more substituents which can each be independently selected from the group consisting of halo, alkyl, aryl, cycloalkyl, cyano, hydroxy, alkoxy, alkylthio, amino, -NH(alkyl), -NH(cycloalkyl), - $N(alkyl)_2$, carboxy and

- C(O)O-alkyl. Non-limiting examples of suitable alkyl groups include methyl, ethyl, n-propyl, isopropyl, n-butyl, and t-butyl, isobutyl, sec-butyl, n-pentyl, n-hexyl, and the like.

"Alkenyl" means an aliphatic hydrocarbon group containing at least one carbon-carbon double bond and which can be straight or branched. $(C_2-C_6)$alkenyl means an alkenyl group that is 2-6 carbon atoms long. The term "substituted alkenyl" means that the alkenyl group can be substituted by one or more substituents which can be the same or different, each substituent being independently selected from the group consisting of halo, alkyl, aryl, cycloalkyl, cyano, and alkoxy. Non-limiting examples of suitable alkenyl groups include ethenyl, propenyl, and n-butenyl..

"Alkynyl" means an aliphatic hydrocarbon group containing at least one carbon-carbon triple bond and which can be straight or branched. Branched means that one or more lower alkyl groups such as methyl, ethyl or propyl, are attached to a linear alkynyl chain. $(C_2-C_6)$alkynyl means an alkynyl group that is 2-6 carbon atoms long. Non-limiting examples of suitable alkynyl groups include ethynyl, propynyl, and 2-butynyl.

The term "substituted alkynyl" means that the alkynyl group can be substituted by one or more substituents each being independently selected from the group consisting of alkyl, aryl and cycloalkyl.

"Amino" means an $-NH_2$ group whilst the term arylamino comprises any group -NH-aryl, wherein aryl is as defined below.

"Halogen" means a fluorine, chlorine, bromine or iodine atom.

With the term aryl, the present invention contemplates any carbocyclic or heterocyclic hydrocarbon with from 1 to 2 ring moieties, either fused or linked to each other by single bonds, wherein at least one of the rings is aromatic. If present, any aromatic heterocyclic hydrocarbon also referred to as heteroaryl group, comprises a 5 to 6 membered ring with from 1 to 3 heteroatoms selected among N, O or S.

The aryl group can be unsubstituted or substituted on the ring with one or more substituents, each being

independently selected from the group consisting of alkyl, aryl, $OCF_3$, OCOalkyl, OCOaryl, $CF_3$, heteroaryl, aralkyl, alkylaryl, heteroaralkyl, alkylheteroaryl, hydroxy, hydroxyalkyl, alkoxy, aryloxy, aralkoxy, acyl, aryl, halo, haloalkyl, haloalkoxy, nitro, cyano, carboxy, alkoxycarbonyl, aryloxycarbonyl, aralkoxycarbonyl, alkylsulfonyl, arylsulfonyl, heteroarylsulfonyl, alkylsulfinyl, arylsulfinyl, heteroarylsulfinyl, alkylthio, arylthio, heteroarylthio, aralkylthio, heteroaralkylthio, cycloalkyl, heterocyclyl, heterocyclenyl, -NH(alkyl), - NH(cycloalkyl), and $-N(alkyl)_2$. Non-limiting examples of suitable aryl groups include phenyl and naphthyl. The "aryl" group can also be substituted by linking two adjacent carbons on its aromatic ring via a combination of one or more carbon atoms and one or more oxygen atoms such as, for example, methylenedioxy, ethylenedioxy, and the like. Examples of aryl groups according to the invention are, for instance, phenyl, biphenyl, α- or β-naphthyl, dihydronaphthyl, thienyl, benzothienyl, furyl, benzofuranyl, pyrrolyl, imidazolyl, pyrazolyl, thiazolyl, isothiazolyl, oxazolyl, isoxazolyl, pyridyl, pyrazinyl, pyrimidinyl, pyridazinyl, indolyl, isoindolyl, purinyl, quinolyl, isoquinolyl, dihydroquinolinyl, quinoxalinyl, benzodioxolyl, indanyl, indenyl, triazolyl, and the like. "Cycloalkyl" means a non-aromatic mono- or multicyclic ring system. $(C_3\text{-}C_6)$cycloalkyl means a cycloalkyl group that is 3-6 carbon atoms long. The cycloalkyl can be optionally substituted on the ring by replacing an available hydrogen on the ring by one or more substituents, each being independently selected from the group consisting of alkyl, aryl, heteroaryl, aralkyl, alkylaryl, aralkenyl, heteroaralkyl, alkylheteroaryl, heteroaralkenyl, hydroxy, hydroxyalkyl, alkoxy, aryloxy, aralkoxy, acyl, aryl, halo, nitro, cyano, carboxy, alkoxycarbonyl, aryloxycarbonyl, aralkoxycarbonyl, alkylsulfonyl, arylsulfonyl, heteroarylsulfonyl, alkylsulfinyl, arylsulfinyl, heteroarylsulfinyl, alkylthio, arylthio, heteroarylthio, aralkylthio, heteroaralkylthio, cycloalkyl, cycloalkenyl, heterocyclyl, heterocyclenyl, -NH(alkyl), - NH(cycloalkyl), and $-N(alkyl)_2$. Non-limiting examples of suitable monocyclic cycloalkyls include cyclopropyl, cyclobutyl, cyclopentyl and cyclohexyl.

"Heterocycle" means any 5 or 6 membered heterocyclic ring comprising from 1 to 3 heteroatoms selected among N, O or S. If the said heterocycle or heterocyclyl group is an aromatic heterocycle, also referred to as heteroaryl, it is encompassed by the above definition given to aryl groups.

As such, besides the above aromatic heterocycles, the term heterocyclyl also encompasses saturated or partially unsaturated heterocycles such as, for instance, pyrroline, pyrrolidine, imidazoline, imidazolidine, pyrazoline, pyrazolidine, piperidine, piperazine, morpholine, and the like.

When the aryl or heteroaryl group is optionally substituted, the substituents are preferably selected from alkyl, haloalkyl, polyfluoroalkyl, hydroxyalkyl, aminoalkyl, amino, alkylamino, dialkylamino, cyano, hydroxy, alkoxy, halogen, as herein defined. Pharmaceutically acceptable salts of the compounds of formula (I) include the acid addition salts with inorganic or organic acids such as, for instance, nitric, hydrochloric, hydrobromic, sulfuric, perchloric, phosphoric, acetic, trifluoroacetic, propionic, glycolic, lactic, oxalic, malonic, malic, maleic, tartaric, citric, benzoic, cinnamic, mandelic, methanesulphonic, isethionic and salicylic acid.

The term "prodrug", as employed herein, denotes a compound that is a drug precursor, which, upon administration to a subject, undergoes chemical conversion by metabolic or chemical processes to yield a compound of formula (I) or a salt and/or solvate thereof. A discussion of prodrugs is provided in T. Higuchi and V. Stella, Pro-drugs as Novel Delivery Systems (1987) Volume 14 of the A.C.S. Symposium Series, and in Bioreversible Carriers in Drug Design, (1987) Edward B. Roche, ed., American Pharmaceutical Association and Pergamon Press, both of which are incorporated herein by reference thereto.

"Solvate" means a physical association of a compound of this invention with one or more solvent molecules. This physical association involves varying degrees of ionic and covalent bonding, including hydrogen bonding. In certain instances the solvate will be capable of isolation, for example when one or more solvent molecules are incorporated in the crystal lattice of the crystalline solid. "Solvate" encompasses both solution-phase and isolatable solvates. Non-limiting examples of suitable solvates include ethanolates, methanolates, and the like. "Hydrate" is a solvate wherein the solvent molecule is $H_2O$. When any variable (e.g., aryl, alkyl, etc.) occurs more than one time in any constituent or in Formula (I), its definition on each occurrence is independent of its definition at every other occurrence. Also, combinations of substituents and/or variables are permissible only if such combinations result in stable compounds.

The following schemes, which are described hereinbelow, further depict how to obtain the compounds of formula (I):

**Scheme I**

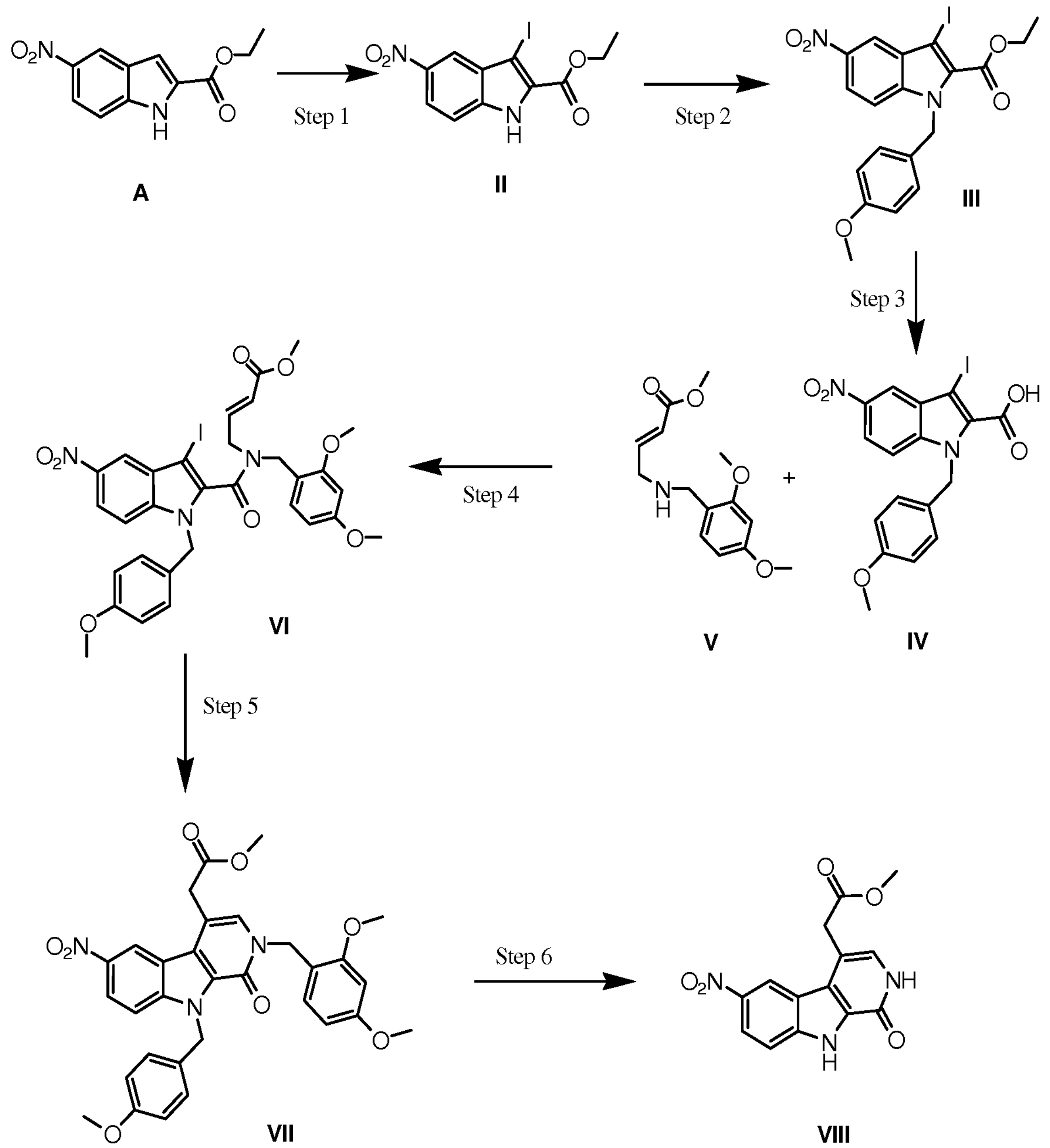


One skilled in the art can easily modify the above schemes based on Maklakov et al. Chem Heterocycl compd 2002, 38(5), 539-542, to make urea derivatives wherein R is $-CH_2OCOR^2$ and $R^1$ is $-NHCONR^4R^5$. In addition, one skilled in the art can modify the above schemes by know methods according to Tois et al. Tetrahedron Lett. 2000, 41(14), 2443-2446 to make derivatives when R is $-CH_2OCOR^2$ and $R^1$ is $-NHR^4$.

## Example 1

**Compound II: ethyl 3-iodo-5-nitro-1H-indole-2-carboxylate**

To Indole **A** (2.56 g, 10.9 mmol, 1 eq) dissolved in DMF (25 ml) KOH (2.3 g, 41.5 mmol, 3.8 eq) was added and stirred at room temperature for 5 min. $I_2$ (2.77 g, 10.9 mmol, 1 eq) dissolved in DMF (10 ml) was added and the solution was stirred at room temperature for 30 min. The reaction mixture was dropped into a solution whose composition was the following: 1.28 g of $NaHSO_3$, 12.8 ml $NH_3$ 28% and 190 ml of water. The precipitate was isolated by filtration and recrystallized with EtOH (450 ml) to yield the title compound **II**. Yield = 3 g (76%).
1H NMR (400 MHz, DMSO-D6) $\delta$ ppm 1.38 (t, 3 H) 4.39 (q, 2 H) 7.65 (q, 1 H) 8.16 (dd, 1 H) 8.31 (d, 1 H) 12.89 (s, 1 H).

## Example 2

### Compound III: ethyl 3-iodo-1-(4-methoxybenzyl)-5-nitro-1H-indole-2-carboxylate

NaH 60% (183 mg, 4.58 mmol, 1.1 eq) was added to compound **II** (1.5 g, 4.17 mmol, 1 eq) dissolved in DMF dry (42 ml), and stirred until hydrogen evolution ended. 4-methoxybenzyl chloride (0.735 ml, 5.42 mmol, 1.3 eq) was added and the reaction mixture was stirred at 25°C for about 16 h. Volatiles were removed and the residue was dissolved in AcOEt and water. The phases were separated and the aqueous one was extracted twice with AcOEt. The organic phases were collected, dried with $Na_2SO_4$ and organic solvent was evaporated to yield the title compound **III** as a crude product. The crude product was used in the next step without purification.

## Example 3

### Compound IV: 3-iodo-1-(4-methoxybenzyl)-5-nitro-1H-indole-2-carboxylic acid

The compound **III** (2 g, 4.17 mmol, 1 eq) was suspended in $H_2O$/THF 1:4 (50 ml) and LiOH*$H_2O$ (256 mg, 6.25 mmol, 1.5 eq) was added; the reaction mixture was then stirred for about 16h. Volatiles were evaporated and the residue was treated with acetic acid until pH=5-5.5 was reached. The yellow precipitate was filtrated and dried in an oven to yield the title compound **IV**.
Yield over 2 steps = 1.03 g (55%).
1H NMR (400 MHz, DMSO-D6) $\delta$ ppm 5.81 (s, 3 H) 6.80 (d, J=8.78 Hz, 2 H) 7.10 (d, J=8.66 Hz, 2 H) 7.72 (d, J=9.15 Hz, 1 H) 8.06 (dd, 1 H) 8.16 - 8.30 (m, 1 H) 11.91 (s, 1 H).

## Example 4

### Compound V: methyl 4-[(2,4-dimethoxybenzyl)amino]but-2-enoate

(E)-4-Bromo-but-2-enoic acid methyl ester (0.5 ml, 4.18 mmol, 1 eq) was added to a solution of 2,4-Dimethoxybenzylamine (1.26 ml, 8.37 mmol, 2 eq) in DCM (10 ml), then stirred for 16h at 25°C. The reaction mixture was quenched with water (20 ml), and the phases were separated. The aqueous phase was extracted twice with DCM. The organic phases were combined, dried over $Na_2SO_4$, filtered, and concentrated under reduced pressure. The crude product was purified by flash chromatography on silica gel using Hexane/AcOEt 1:1 as eluent. The title compound **V** was isolated as yellow oil that is preferably reacted when freshly prepared. Yield: 150 mg (13%).
1H NMR (300 MHz, CDCl3) $\delta$ ppm 2.63 (m, 2 H) 2.95-3.1 (m, 8 H) 5.4 (bd, 2 H) 5.65-5.7 (m, 2 H) 6.25 (m,1 H) 6.35 (m, 1 H).

## Example 5

### Compound VI: methyl 4-[(2,4-dimethoxybenzyl){[3-iodo-1-(4-methoxybenzyl)-5-nitro-1H-indol-2-yl]carbonyl}amino]but-2-enoate

In a round-bottomed flask were placed EDC (102 mg, 0.53 mmol, 2 eq) and HOBt (81 mg, 0.53 mmol, 2 eq) and DCM (4 ml) was added. The suspension was stirred at 0°C until complete dissolution was achieved, then a solution of **IV** (120 mg, 0.265 mmol, 1 eq) in DMF (1 ml) was added, the resulting mixture was stirred at 0°C. After 20 min the amine **V** (140 mg, 0.53 mmol, 2 eq) dissolved in DCM (6 ml) was added. After 2 h the reaction was allowed to reach r.t. and stirred for 16 h. The reaction mixture was then quenched with $H_2O$, extracted with DCM and the organic phase was washed with saturated $NaHCO_3$ aqueous solution, and brine. It was then dried over $Na_2SO_4$, filtered, and concentrated under reduced pressure. The crude product was purified by flash chromatography on silica gel using Hexane/AcOEt 6:4 as eluent to yield the title compound **VI**. Yield = 99 mg (53%).
HPLC Rt = 8.8 min; [M+H]+ =700. 1H NMR (400 MHz, DMSO-D6) $\delta$ ppm 3.63 - 3.76 (m, 2 H) 3.78 - 3.80 (m, 1 H) 3.85 - 3.90 (m, 1 H) 4.02 - 4.10 (m, 1 H) 4.32 - 4.49 (m, 3 H) 4.63 (dd, J=62.68, 14.39 Hz, 1 H) 5.22 - 5.44 (m, 3 H) 5.67 (td, J=15.70, 1.48 Hz, 1 H) 6.00 (dt, J=15.82, 1.60 Hz, 1 H) 6.33 (dd, J=8.41, 2.44 Hz, 2 H) 6.44 (t, 1 H) 6.47 - 6.51 (m, 1 H) 6.54 (dd, J=8.35, 2.38 Hz, 1 H) 6.58 (d, J=2.44 Hz, 1 H) 6.73 (d, J=8.41 Hz, 1 H) 6.78 (t, J=5.61 Hz, 1 H) 6.83 (dq, 1 H) 7.06 - 7.22 (m, 3 H) 7.32 (d, J=8.29 Hz, 1 H) 7.72 (dd, J=25.91, 9.21 Hz, 1 H) 8.13 (dd, J=9.15, 2.32 Hz, 1 H) 8.26 (dd, J=19.08, 2.13 Hz, 1 H).

**Example 6**

**Compound VII: methyl [2-(2,4-dimethoxybenzyl)-9-(4-methoxybenzyl)-6-nitro-1-oxo-2,9-dihydro-1H-beta-carbolin-4-yl]acetate**

A suspension of $PPh_3$ (74.2 mg, 0.283 mmol, 2 eq), palladium acetate (32 mg, 0.142 mmol, 1 eq) and silver carbonate (78 mg, 0.283 mmol, 2 eq) in dry THF (2.5 ml) was stirred for 15 minutes at 25°C. A solution of compound **VI** (99 mg, 0.142 mmol, 1 eq) in dry THF (2.5 ml) was added to the reaction mixture that was stirred at r.t. for 16 h. The palladium and the silver salts were removed by filtration through a celite pad and the crude product was purified by flash chromatography on silica gel using Hexane/AcOEt 8:2 as eluent to yield the title compound **VII**. Yield = 33 mg (43%).
1H-NMR (500 MHz, $CDCl_3$) δ ppm 6.46 (m, 2 H) 6.78 (d, J=8.55 Hz, 2 H) 7.17 (d, J=8.55 Hz, 1 H) 7.25 (d, J=4.64 Hz, 1 H) 7.31 (d, J=8.30 Hz, 1 H) 7.49 (d, J=9.28 Hz, 1 H) 8.28 (dd, J=9.28, 1.95 Hz, 1 H) 8.95 (d, J=1.71 Hz, 1 H).

**Example 7**

**Compound VIII: methyl (6-nitro-1-oxo-2,9-dihydro-1H-beta-carbolin-4-yl)acetate**

Trifluoroacetic acid (500 ml) was added to a solution of compound **VII** (15.0 g, 26.0 mmol) dissolved in anisol (150 ml) and stirred at 80°C for 8h. The reaction was quenched with saturated $NaHCO_3$ aqueous solution and extracted with DCM. The aqueous phase was extracted twice with DCM. The organic phases were combined, dried over $Na_2SO_4$, filtered, and concentrated under reduced pressure. The crude product was purified by preparative HPLC to yield the title compound **VIII**. Yield = 3 g (40 %).
1H NMR (500 MHz, DMSO-D6) δ ppm 4.08 (s, 2 H) 7.14 (d, J=5.75 Hz, 1 H) 7.66 (d, J=9.16 Hz, 1 H) 8.27 (dd, J=9.16, 1.70 Hz, 1 H) 8.82 (d, J=1.92 Hz, 1 H) 11.68 (d, J=4.47 Hz, 1 H) 12.90 (bs, 1H).

**Scheme 2**

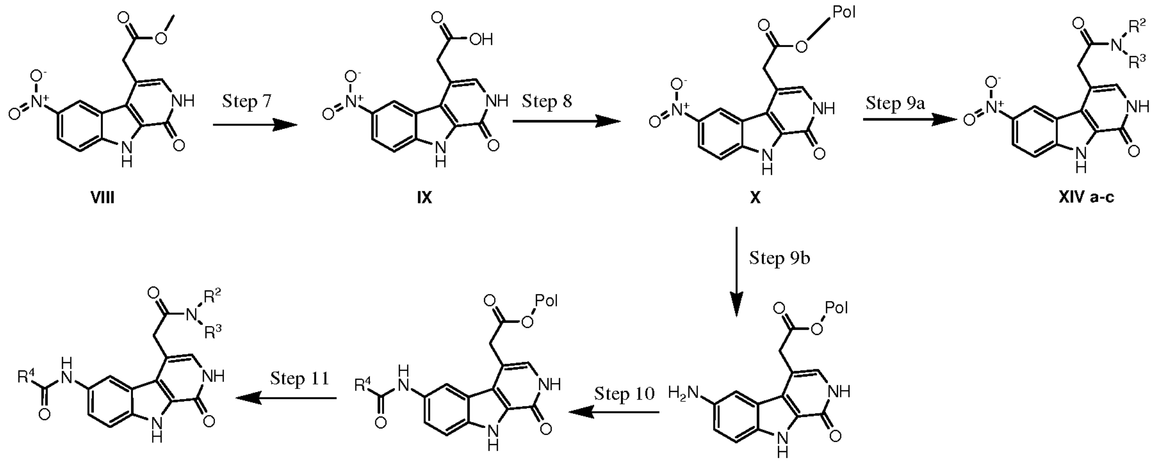


VIII IX X XIV a-c

XIII a-q XII a-q XI

$R^2$, $R^3$ and $R^4$ carry the same meaning as in the compound of formula (I) described herein.

**Example 8**

**Compound IX: (6-Nitro-1-oxo-2,9-dihydro-1H-beta-carbolin-4-yl)acetic acid**

LiOH*$H_2O$ (83.7 mg, 1.993 mmol, 3 eq) was added to suspension of compound **VIII** (200 mg, 0.664 mmol, 1 eq) in $H_2O$/MeOH/THF 1:1:4 (6 ml), was stirred for 2h at 25°C. The reaction mixture was cooled to 0°C and quenched with HCl 23% until the solution reached pH 5. Organic volatiles were removed under reduced pressure and the yellow residue was filtered and dried to give pure compound IX. Yield = 170 mg (90%), 1H NMR (400 MHz, DMSO-D6) δ ppm 3.97 (s, 1 H) 7.13 (d, J=5.98 Hz, 1 H) 7.70 (d, J=9.15 Hz, 1 H) 8.30 (dd, J=9.15, 2.32 Hz, 1 H) 8.92 (d, J=2.20 Hz, 1 H) 11.65 (d, J=6.10 Hz, 1 H) 12.84 (s, 1 H).

**Example 9**

**Compound X: (6-Nitro-1-oxo-2,9-dihydro-1H-beta-carbolin-4-yl)-acetic acid HMBA-AM resin ester**

DIC (163.4 mg, 1.05 mmol, 3 eq) and DMAP (12.8 mg, 0.105 mmol, 0.3 eq) were added to a solution of **IX** (120 mg, 0.418 mmol, 1.2 eq) in DCM/DMA 1:1 (10 ml) cooled to 0°C, the reaction mixture was stirred for 30 minutes then added to HMBA-AM resin (4-hydroxymethylbenzoic acid polymer bound (aminomethyl-copolystyrene-1%dvb) (0.458 g, 0.76 mmol/g, 0.348 mmol, 1 eq) and the final suspension was shaken overnight. The resin was rinsed with DCM 2×, DMA 2×, DCM 2×, DMA 2× and DCM 3×. The resin **X** was used in the next step.

**Example 10**

**Compound XI: (6-Amino-1-oxo-2,9-dihydro-1H-beta-carbolin-4-yl)-acetic acid HMBA-AM resin ester**

$SnCl_2$ (474 mg, 2.50 mmol, 10 eq) was added to a suspension of **X** (0.25g, 1 eq) in DMA (3 ml) and the reaction was shaken for 48 h at 25°C. The resin was rinsed with DMA 2x, THF 2×, DCM 2×, DMA 2×, DCM 3× and dried under nitrogen flux. The resin **XI** was used in the next step.

**Example 11**

**Compound XIIa: ({1-Oxo-6-[(thiophene-2-carbonyl)-amino]-2,9-dihydro-1H-beta-carbolin-4-yl}-acetic acid HMBA-AM resin ester**

Thiophene-2-carboxylic acid (0.24 mmol, 3 eq), PyBOP (125 mg, 0.24 mmol, 3 eq) and DIPEA (41.0 ul, 0.24 mmol, 3 eq) were added to a suspension of resin **XI** (0.08 mmol, 1 eq) in dry DMA (3 ml). The resulting suspension was shaken for 16 h at 25°C. The resin was rinsed with DMA 3×, THF 2×, DMA 2×, THF 2× and DCM 3×. The title compound resin was used in the next step.

**Example 12**

**Compound XIIb: {1-Oxo-6-[(pyridine-3-carbonyl)-amino]-2,9-dihydro-1H-beta-carbolin-4-yl}-acetic acid HMBA-AM resin ester**

Nicotinic acid (0.24 mmol, 3 eq), PyBOP (125 mg, 0.24 mmol, 3 eq) and DIPEA (41.0 ul, 0.24 mmol, 3 eq) were added to a suspension of resin **XI** (0.08 mmol, 1 eq) in dry DMA (3 ml). The resulting suspension was shaken for 16 h at 25°C. The resin was rinsed with DMA 3x, THF 2×, DMA 2×, THF 2× and DCM 3×. The title compound resin was used in the next step.

**Example 13**

**Compound XIIc: (1-Oxo-6-phenylacetylamino-2,9-dihydro-1H-beta-carbolin-4-yl)-acetic acid HMBA-AM resin ester**

Phenylacetic acid (0.24 mmol, 3 eq), PyBOP (125 mg, 0.24 mmol, 3 eq) and DIPEA (41.0 ul, 0.24 mmol, 3 eq) were added to a suspension of resin **XI** (0.08 mmol, 1 eq) in dry DMA (3 ml). The resulting suspension was shaken for 16 h at 25°C. The title compound resin was rinsed with DMA 3×, THF 2×, DMA 2×, THF 2× and DCM 3×. The title compound resin was used in the next step.

**Example 14**

**Compound XIId: (6-Isobutyrylamino-1-oxo-2,9-dihydro-1H-beta-carbolin-4-yl)-acetic acid HMBA-AM resin ester**

2-Methylpropanoic acid (0.24 mmol, 3 eq), PyBOP (125 mg, 0.24 mmol, 3 eq) and DIPEA (41.0 ul, 0.24 mmol, 3 eq) were added to a suspension of resin **XI** (0.08 mmol, 1 eq) in dry DMA (3 ml). The resulting suspension was shaken for 16 h at 25°C. The resin was rinsed with DMA 3×, THF 2×, DMA 2×, THF 2× and DCM 3×. The title compound resin was used in the next step.

**Example 15**

**Compound XIIe: {6-[2-(4-Methanesulfonylamino-phenyl)-acetylamino]-1-oxo-2,9-dihydro-1H-beta-carbolin-4-yl}-acetic acid HMBA-AM resin ester**

{4-[(Methylsulfonyl)amino]phenyl}acetic acid (0.24 mmol, 3 eq), PyBOP (125 mg, 0.24 mmol, 3 eq) and DIPEA (41.0 ul, 0.24 mmol, 3 eq) were added to a suspension of resin **XI** (0.08 mmol, 1 eq) in dry DMA (3 ml). The resulting suspension was shaken for 16 h at 25°C. The resin was rinsed with DMA 3×, THF 2×, DMA 2×, THF 2× and DCM 3x. The title compound resin was used in the next step.

**Example 16**

**Compound XIIf: {6-[2-(3,4-Dimethoxy-phenyl)-acetylamino]-1-oxo-2,9-dihydro-1H-beta-carbolin-4-yl}-acetic acid HMBA-AM resin ester**

(3,4-dimethoxyphenyl)acetic acid (0.24 mmol, 3 eq), PyBOP (125 mg, 0.24 mmol, 3 eq) and DIPEA (41.0 ul, 0.24 mmol, 3 eq) were added to a suspension of resin **XI** (0.08 mmol, 1 eq) in dry DMA (3 ml). The resulting suspension was shaken for 16 h at 25°C. The resin was rinsed with DMA 3×, THF 2×, DMA 2×, THF 2× and DCM 3×. The title compound resin was used in the next step.

**Example 17**

**Compound XIIg: [6-(2,2-Dimethyl-propionylamino)-1-oxo-2,9-dihydro-1H-beta-carbolin-4-yl]-acetic acid HMBA-AM resin ester**

Pivalic acid (0.24 mmol, 3 eq), PyBOP (125 mg, 0.24 mmol, 3 eq) and DIPEA (41.0 ul, 0.24 mmol, 3 eq) were added to a suspension of resin **XI** (0.08 mmol, 1 eq) in dry DMA (3 ml). The resulting suspension was shaken for 16 h at 25°C. The resin was rinsed with DMA 3x, THF 2×, DMA 2×, THF 2× and DCM 3×. The title compound resin was used in the next step.

**Example 18**

**Compound XIIh: [6-(2-Methyl-hexanoylamino)-1-oxo-2,9-dihydro-1H-beta-carbolin-4-yl]-acetic acid HMBA-AM resin ester**

2-Methylhexanoic acid (0.24 mmol, 3 eq), PyBOP (125 mg, 0.24 mmol, 3 eq) and DIPEA (41.0 ul, 0.24 mmol, 3 eq) were added to a suspension of resin **XI** (0.08 mmol, 1 eq) in dry DMA (3 ml). The resulting suspension was shaken for 16 h at 25°C. The resin was rinsed with DMA 3×, THF 2×, DMA 2×, THF 2× and DCM 3×. The title compound resin was used in the next step.

**Example 19**

**Compound XIIi: {6-[(1-Methyl-1H-pyrrole-2-carbonyl)-amino]-1-oxo-2,9-dihydro-1H-beta-carbolin-4-yl}-acetic acid HMBA-AM resin ester**

1-Methyl-1H-pyrrole-2-carboxylic acid (0.24 mmol, 3 eq), PyBOP (125 mg, 0.24 mmol, 3 eq) and DIPEA (41.0 ul, 0.24 mmol, 3 eq) were added to a suspension of resin **XI** (0.08 mmol, 1 eq) in dry DMA (3 ml). The resulting suspension was shaken for 16 h at 25°C. The resin was rinsed with DMA 3×, THF 2×, DMA 2×, THF 2× and DCM 3×. The title compound resin was used in the next step.

**Example 20**

**Compound XIIl: {6-[2-(2-Methoxy-phenyl)-acetylamino]-1-oxo-2,9-dihydro-1H-beta-carbolin-4-yl}-acetic acid HMBA-AM resin ester**

(2-Methoxyphenyl)acetic acid (0.24 mmol, 3 eq), PyBOP (125 mg, 0.24 mmol, 3 eq) and DIPEA (41.0 ul, 0.24 mmol, 3 eq) were added to a suspension of resin **XI** (0.08 mmol, 1 eq) in dry DMA (3 ml). The resulting suspension was shaken for 16 h at 25°C. The resin was rinsed with DMA 3×, THF 2×, DMA 2×, THF 2× and DCM 3×. The title

compound resin was used in the next step.

**Example 21**

**Compound XIIm: {1-Oxo-6-[(tetrahydro-furan-2-carbonyl)-amino]-2,9-dihydro-1H-beta-carbolin-4-yl}-acetic acid HMBA-AM resin ester**

Tetrahydrofuran-2-carboxylic acid (0.24 mmol, 3 eq), PyBOP (125 mg, 0.24 mmol, 3 eq) and DIPEA (41.0 ul, 0.24 mmol, 3 eq) were added to a suspension of resin **XI** (0.08 mmol, 1 eq) in dry DMA (3 ml). The resulting suspension was shaken for 16 h at 25°C.

The resin was rinsed with DMA 3×, THF 2×, DMA 2×, THF 2× and DCM 3×. The title compound resin was used in the next step.

**Example 22**

**Compound XIIn: [1-Oxo-6-(2-m-tolyl-acetylamino)-2,9-dihydro-1H-beta-carbolin-4-yl]-acetic acid HMBA-AM resin ester**

(3-Methylphenyl)acetic acid (0.24 mmol, 3 eq), PyBOP (125 mg, 0.24 mmol, 3 eq) and DIPEA (41.0 ul, 0.24 mmol, 3 eq) were added to a suspension of resin **XI** (0.08 mmol, 1 eq) in dry DMA (3 ml). The resulting suspension was shaken for 16 h at 25°C. The resin was rinsed with DMA 3×, THF 2×, DMA 2×, THF 2× and DCM 3×. The title compound resin was used in the next step.

**Example 23**

**Compound XIIo: {6-[(5-Methyl-pyrazine-2-carbonyl)-amino]-1-oxo-2,9-dihydro-1H-beta-carbolin-4-yl}-acetic acid HMBA-AM resin ester**

5-Methylpyrazine-2-carboxylic acid (0.24 mmol, 3 eq), PyBOP (125 mg, 0.24 mmol, 3 eq) and DIPEA (41.0 ul, 0.24 mmol, 3 eq) were added to a suspension of resin **XI** (0.08 mmol, 1 eq) in dry DMA (3 ml). The resulting suspension was shaken for 16 h at 25°C. The resin was rinsed with DMA 3×, THF 2×, DMA 2×, THF 2× and DCM 3×. The title compound resin was used in the next step.

**Example 24**

**Compound XIIp: {6-[(5-Bromo-furan-2-carbonyl)-amino]-1-oxo-2,9-dihydro-1H-beta-carbolin-4-yl}-acetic acid HMBA-AM resin ester**

5-Bromo-2-furoic acid (0.24 mmol, 3 eq), PyBOP (125 mg, 0.24 mmol, 3 eq) and DIPEA (41.0 ul, 0.24 mmol, 3 eq) were added to a suspension of resin **XI** (0.08 mmol, 1 eq) in dry DMA (3 ml). The resulting suspension was shaken for 16 h at 25°C. The resin was rinsed with DMA 3×, THF 2×, DMA 2×, THF 2× and DCM 3×. The title compound resin was used in the next step.

**Example 25**

**Compound XIIq: {1-Oxo-6-[2-(pyridin-4-ylsulfanyl)-acetylamino]-2,9-dihydro-1H-beta-carbolin-4-yl}-acetic acid HMBA-AM resin ester**

(Pyridin-4-ylthio)acetic acid (0.24 mmol, 3 eq), PyBOP (125 mg, 0.24 mmol, 3 eq) and DIPEA (41.0 ul, 0.24 mmol, 3 eq) were added to a suspension of resin **XI** (0.08 mmol, 1 eq) in dry DMA (3 ml). The resulting suspension was shaken for 16 h at 25°C. The resin was rinsed with DMA 3×, THF 2×, DMA 2×, THF 2× and DCM 3×. The title compound resin was used in the next step.

**Example 26**

**Compound XIIIa: N-{4-[2-(isopropylamino)-2-oxoethyl]-1-oxo-2,9-dihydro-1H-beta-carbolin-6-yl}thiophene-2-carboxamide**

The resin **XIIa** (0.08 mmol) was suspended in isopropylamine (2 ml) and shaken for 16 h at 25°C. The solution was collected, dried and the residue was purified by preparative HPLC-MS to afford the title compound **XIIIa.**
HPLC Rt = 3.51 min; [M+H]+ =409. 1H NMR (500 MHz, DMSO-D6) δ ppm 1.05 (d, J=6.58 Hz, 6 H) 3.71 (s, 2 H) 3.86 (dt, J=20.51, 6.48 Hz, 1 H) 6.88 (d, J=5.76 Hz, 1 H) 7.24 (dd, J=4.94, 3.84 Hz, 1 H) 7.49 - 7.55 (m, 1 H) 7.60 (d, J=8.78 Hz, 1 H) 7.84 (d, J=4.94 Hz, 1 H) 7.95 (d, J=7.68 Hz, 1 H) 8.02 (d, J=3.57 Hz, 1 H) 8.41 (s, 1 H) 10.23 (s, 1 H) 11.30 (d, J=5.49 Hz, 1 H) 11.98 (s, 1 H).
According to the same procedure, applied to resins **XIIb-d,** the following compounds were also obtained:

**Example 27**

**Compound XIIIb: N-{4-[2-(isopropylamino)-2-oxoethyl]-1-oxo-2,9-dihydro-1H-beta-carbolin-6-yl}nicotinamide**

HPLC Rt = 2.5 min; [M+H]+ = 404. 1H NMR (500 MHz, DMSO-D6) δ ppm 1.05 (d, J=6.58 Hz, 6 H) 3.71 (s, 2 H) 3.87 (td, J=13.65, 6.72 Hz, 1 H) 6.88 (s, 1 H) 7.53 (s, 1 H) 7.60 (dd, J=8.09, 4.80 Hz, 1 H) 7.66 (dd, J=8.78, 1.92 Hz, 1 H) 7.95 (d, J=7.68 Hz, 1 H) 8.34 (dt, J=7.75, 1.89 Hz, 1 H) 8.46 (d, J=1.65 Hz, 1 H) 8.78 (dd, J=4.80, 1.51 Hz, 1 H) 9.16 (s, 1 H) 10.44 (s, 1 H) 11.31 (s, 1 H) 12.00 (s, 1 H).

**Example 28**

**Compound XIIIc: N-isopropyl-2-{1-oxo-6-[(phenylacetyl)amino]-2,9-dihydro-1H-beta-carbolin-4-yl}acetamide**

HPLC Rt = 3.67 min; [M+H]+ = 417. 1H NMR (500 MHz, DMSO-D6) δ ppm 1.02 (d, J=6.58 Hz, 6 H) 3.64 - 3.68 (m, 4 H) 3.83 (td, J=13.65, 7.00 Hz, 1 H) 6.84 (s, 1 H) 7.25 (t, J=7.13 Hz, 1 H) 7.30 - 7.39 (m, 4 H) 7.45 (d, 1 H) 7.48 - 7.53 (m, 1 H) 7.87 (d, J=7.68 Hz, 1 H) 8.36 (s, 1 H) 10.11 (s, 1 H) 11.26 (s, 1 H) 11.91 (s, 1 H).

**Example 29**

**Compound XIIId: N-{4-[2-(isopropylamino)-2-oxoethyl]-1-oxo-2,9-dihydro-1H-beta-carbolin-6-yl}-2-methylpropanamide**

HPLC Rt = 2.91 min; [M+H]+ = 370. 1H NMR (500 MHz, DMSO-D6) δ ppm 1.06 (d, J=6.58 Hz, 6 H) 1.12 (d, J=6.86 Hz, 6 H) 2.60 (dt, J=13.44, 6.72 Hz, 1 H) 3.67 (s, 2 H) 3.85 (ddd, J=20.57, 6.58, 6.31 Hz, 1 H) 6.83 (s, 1 H) 7.42 (d, 1 H) 7.44 - 7.47 (m, 1 H) 7.90 (d, J=7.96 Hz, 1 H) 8.38 (d, J=1.37 Hz, 1 H) 9.74 (s, 1 H) 11.25 (s, 1 H) 11.88 (s, 1 H).

**Example 30**

**Compound XIIIe: N-{4-[2-(methylamino)-2-oxoethyl]-1-oxo-2,9-dihydro-1H-beta-carbolin-6-yl}-2-{4-[(methylsulfonyl)amino]phenyl}acetamide**

The resin **XIIe** (0.08 mmol) was suspended in a 2M solution of $MeNH_2$ in THF (3 ml) and shaken for 16 h at 25°C. The solution was collected, dried and the residue was purified by preparative HPLC-MS to afford the title compound **XIIIe.**
HPLC Rt = 2.59 min; [M+H]+ = 482. 1H NMR (500 MHz, DMSO-D6) δ ppm 2.95 (s, 3 H) 3.60 - 3.63 (m, 2 H) 3.69 (s, 2 H) 6.87 (d, J=6.03 Hz, 1 H) 7.17 (d, J=7.96 Hz, 2 H) 7.33 (d, J=7.96 Hz, 2 H) 7.46 (d, J=8.78 Hz, 1 H) 7.54 (dd, J=8.78, 1.92 Hz, 1 H) 7.77 (s, 1 H) 8.27 (s, 1 H) 10.12 (s, 1 H) 11.30 (s, 1 H) 11.92 (s, 1 H).
According to the same procedure, applied to resins **XIIf-q,** the following compounds were also obtained:

**Example 31**

**Compound XIIIf: 2-(3,4-Dimethoxyphenyl)-N-{4-[2-(methylamino)-2-oxoethyl]-1-oxo-2,9-dihydro-1H-beta-carbolin-6-yl}acetamide**

HPLC Rt = 3.45 min; $[M+H]^+$ = 449. 1H NMR (400 MHz, DMSO-D6) δ ppm 2.57 (d, J=4.63 Hz, 3 H) 3.58 (s, 2

H) 3.69 (s, 2 H) 3.74 (s, 3 H) 3.78 (s, 3 H) 6.83 - 6.95 (m, 3 H) 7.00 (d, J=1.71 Hz, 1 H) 7.45 (d, J=8.78 Hz, 1 H) 7.53 - 7.58 (m, 1 H) 7.74 (d, J=4.63 Hz, 1 H) 8.27 (s, 1 H) 10.04 (s, 1 H) 11.29 (d, J=5.85 Hz, 1 H) 11.91 (s, 1 H).

## Example 32

### Compoune XIIIg: 2,2-Dimethyl-N-{4-[2-(methylamino)-2-oxoethyl]-1-oxo-2,9-dihydro-1H-beta-carbolin-6-yl}propanamide

HPLC Rt = 3.32 min; [M+H]+ = 355. 1H NMR (400 MHz, DMSO-D6) δ ppm 1.27 (s, 9 H) 2.61 (d, J=4.63 Hz, 3 H) 3.72 (s, 2 H) 6.87 (d, J=5.73 Hz, 1 H) 7.45 (d, J=8.90 Hz, 1 H) 7.55 (dd, J=8.90, 1.95 Hz, 1 H) 7.84 (q, J=4.55 Hz, 1 H) 8.24 (s, 1 H) 9.17 (s, 1 H) 11.28 (d, J=5.37 Hz, 1 H) 11.90 (s, 1 H).

## Example 33

### Compound XIIIh: 2-Methyl-N-{4-[2-(methylamino)-2-oxoethyl]-1-oxo-2,9-dihydro-1H-beta-carbolin-6-yl}hexanamide

HPLC Rt = 4.12 min; [M+H]+ = 383. 1H NMR (500 MHz, DMSO-D6) δ ppm 1.10 (d, J=6.89 Hz, 3 H) 1.55 - 1.69 (m, 2 H) 2.47 - 2.51 (m, 1 H) 2.57 (d, J=4.53 Hz, 3 H) 3.68 (s, 2 H) 6.84 (d, J=6.10 Hz, 1 H) 7.42 (d, J=8.66 Hz, 1 H) 7.48 - 7.55 (m, 1 H) 7.74 - 7.90 (m, 1 H) 8.29 (s, 1 H) 8.53 (s, 1 H) 9.77 (s, 1 H) 11.27 (s, 1 H) 11.89 (s, 1 H).

## Example 34

### Compound XIIIi: 1-Methyl-N-{4-[2-(methylamino)-2-oxoethyl]-1-oxo-2,9-dihydro-1H-beta-carbolin-6-yl}-1H-pyrrole-2-carboxamide

HPLC Rt = 3.55 min; [M+H]+ = 378. 1H NMR (400 MHz, DMSO-D6) δ ppm 2.61 (d, J=4.63 Hz, 3 H) 3.73 (s, 2 H) 3.92 (s, 3 H) 6.12 (dd, J=3.90, 2.56 Hz, 1 H) 6.89 (d, J=5.73 Hz, 1 H) 6.99 - 7.01 (m, 1 H) 7.04 (dd, J=3.96, 1.77 Hz, 1 H) 7.49 (d, J=8.78 Hz, 1 H) 7.63 (dd, J=8.90, 1.95 Hz, 1 H) 7.80 (s, 1 H) 8.30 (s, 1 H) 9.74 (s, 1 H) 11.29 (d, J=5.73 Hz, 1 H) 11.92 (s, 1 H).

## Example 35

### Compound XIIIl: 2-(2-Methoxyphenyl)-N-{4-[2-(methylamino)-2-oxoethyl]-1-oxo-2,9-dihydro-1H-beta-carbolin-6-yl}acetamide

HPLC Rt = 3.76 min; [M+H]+ = 419. 1H NMR (400 MHz, DMSO-D6) δ ppm 2.56 (d, J=4.63 Hz, 3 H) 3.66 (s, 2 H) 3.69 (s, 2 H) 3.81 (s, 3 H) 6.87 (d, J=5.73 Hz, 1 H) 6.92 (td, J=7.41, 1.04 Hz, 1 H) 7.01 (d, J=7.80 Hz, 1 H) 7.22 - 7.30 (m, 2 H) 7.47 (d, J=8.78 Hz, 1 H) 7.55 (dd, J=8.84, 1.89 Hz, 1 H) 7.75 (d, J=4.76 Hz, 1 H) 8.29 (s, 1 H) 9.97 (s, 1 H) 11.29 (d, J=5.61 Hz, 1 H) 11.89 (s, 1 H).

## Example 36

### Compound XIIIm: N-{4-[2-(methylamino)-2-oxoethyl]-1-oxo-2,9-dihydro-1H-beta-carbolin-6-yl}tetrahydrofuran-2-carboxamide

HPLC Rt = 2.79 min; [M+H]+ = 369. 1H NMR (400 MHz, DMSO-D6) δ ppm 1.91 (q, J=6.71 Hz, 2 H) 1.98 - 2.31 (m, 2 H) 2.60 (d, J=4.63 Hz, 3 H) 3.71 (s, 2 H) 3.95 (m, 2 H) 4.43 (dd, J=8.23, 5.55 Hz, 1 H) 6.88 (d, J=5.85 Hz, 1 H) 7.47 (d, J=8.90 Hz, 1 H) 7.67 (dd, J=8.96, 2.01 Hz, 1 H) 7.78 - 7.87 (m, 1 H) 8.28 (s, 1 H) 9.57 (s, 1 H) 11.29 (d, J=5.73 Hz, 1 H) 11.93 (s, 1 H).

## Example 37

### Compound XIIIn: N-{4-[2-(methylamino)-2-oxoethyl]-1-oxo-2,9-dihydro-1H-beta-carbolin-6-yl}-2-(3-methylphenyl)acetamide

HPLC Rt = 4.02 min; [M+H]+ = 403. 1H NMR (500 MHz, DMSO-D6) δ ppm 2.31 (s, 3 H) 2.55 (s, 3 H) 3.62 (s, 2 H) 3.66 - 3.71 (m, 2 H) 6.87 (d, J=5.76 Hz, 1 H) 7.08 (d, J=7.41 Hz, 1 H) 7.14 - 7.20 (m, 2 H) 7.22 (t, J=7.54 Hz, 1 H)

7.46 (d, J=8.78 Hz, 1 H) 7.53 (dd, J=8.78, 1.92 Hz, 1 H) 7.77 (s, 1 H) 8.28 (s, 1 H) 10.11 (s, 1 H) 11.31 (d, J=5.49 Hz, 1 H) 11.93 (s, 1 H).

## Example 38

**Compound XIIIo: 5-Methyl-N-{4-[2-(methylamino)-2-oxoethyl]-1-oxo-2,9-dihydro-1H-beta-carbolin-6-yl}pyrazine-2-carboxamide**

HPLC Rt = 3.17 min; [M+H]+ = 391. 1H NMR (500 MHz, DMSO-D6) δ ppm 2.60 (d, J=4.66 Hz, 3 H) 2.66 (s, 3 H) 3.75 (s, 2 H) 6.91 (d, J=5.76 Hz, 1 H) 7.53 (d, J=11.80 Hz, 1 H) 7.84 (d, J=4.66 Hz, 1 H) 7.91 (dd, J=9.05, 1.92 Hz, 1 H) 8.51 (s, 1 H) 8.73 (s, 1 H) 9.20 (s, 1 H) 10.61 (s, 1 H) 11.35 (d, J=5.49 Hz, 1 H) 12.01 (s, 1 H).

## Example 39

**Compound XIIIp: 5-Bromo-N-{4-[2-(methylamino)-2-oxoethyl]-1-oxo-2,9-dihydro-1H-beta-carbolin-6-yl}-2-furamide**

HPLC Rt = 3.79 min; [M+H]+ = 443. 1H NMR (400 MHz, DMSO-D6) δ ppm 2.59 (d, J=4.63 Hz, 3 H) 3.73 (s, 2 H) 6.85 (d, J=3.54 Hz, 1 H) 6.90 (d, J=5.73 Hz, 1 H) 7.36 (d, J=3.54 Hz, 1 H) 7.53 (d, J=11.80 Hz, 1 H)7.65 (dd, J=8.90, 1.95 Hz, 1 H) 7.77 (d, J=4.76 Hz, 1 H) 8.34 (s, 1 H) 10.21 (s, 1 H) 11.32 (d, J=5.73 Hz, 1 H) 11.99 (s, 1 H)

## Example 40

**Compound XIIIq: N-methyl-2-(1-oxo-6-{[(pyridin-4-ylthio)acetyl]amino}-2,9-dihydro-1H-beta-carbolin-4-yl)acetamide**

HPLC Rt = 2.92 min; [M+H]+ = 422. 1H NMR (400 MHz, DMSO-D6) δ ppm 2.56 (d, J=4.63 Hz, 3 H) 3.70 (s, 2 H) 4.05 (s, 2 H) 6.89 (d, J=5.73 Hz, 1 H) 7.37 - 7.42 (m, 2 H) 7.46 - 7.50 (m, 1 H) 7.52 - 7.57 (m, 1 H) 7.74 (d, J=4.63 Hz, 1 H) 8.24 (s, 1 H) 8.41 (d, J=6.10 Hz, 2 H) 10.29 (s, 1 H) 11.30 (d, J=5.37 Hz, 1 H) 11.95 (s, 1 H).

Compounds **XIVa**-c were prepared according to the following procedures:

## Example 41

**Compound XIVa: N-benzyl-2-(6-nitro-1-oxo-2,9-dihydro-1H-beta-carbolin-4-yl)acetamide**

The resin **X** (0.08 mmol) was suspended in benzylamine (2 ml) and shaken for 16 h at 25°C. The solution was collected, dried and the residue was purified by preparative HPLC-MS to afford the title compound **XIVa.**
HPLC Rt = 6.61 min; [M+H]+ = 377. 1H NMR (500 MHz, DMSO-D6) δ ppm 3.88 (s, 2 H) 4.26 (d, J=6.04 Hz, 2 H) 7.11 - 7.14 (m, 1 H) 7.14 - 7.19 (m, 5 H) 7.67 (d, J=9.05 Hz, 1 H) 8.26 (dd, J=9.05, 1.92 Hz, 1 H) 8.54 (t, J=5.90 Hz, 1 H) 9.00 (d, J=2.19 Hz, 1 H) 11.64 (s, 1 H).

## Example 42

**Compound XIVb: N-isopropyl-2-(6-nitro-1-oxo-2,9-dihydro-1H-beta-carbolin-4-yl)acetamide**

The resin **X** (0.08 mmol) was suspended in isopropylamine (2 ml) and shaken for 16 h at 25°C. The solution was collected, dried and the residue was purified by preparative HPLC-MS to afford the title compound **XIVb.**
HPLC Rt = 5.66 min; [M+H]+ = 329. 1H NMR (500 MHz, DMSO-D6) δ ppm 1.17 (d, J=6.58 Hz, 6 H) 3.78 (s, 2 H) 3.83 (q, 1 H) 7.67 (d, J=9.05 Hz, 1 H) 8.15 (d, J=7.41 Hz, 1 H) 8.30 (dd, J=9.05, 2.19 Hz, 1 H) 8.97 (d, J=2.19 Hz, 1 H).

## Example 43

**Compound XIVc: N-methyl-2-(6-nitro-1-oxo-2,9-dihydro-1H-beta-carbolin-4-yl)acetamide**

The resin **X** (0.08 mmol) was suspended in a 2M solution of $MeNH_2$ in THF (3 ml) and shaken for 16 h at 25°C. The solution was collected, dried and the residue was purified by preparative HPLC-MS to afford the title compound **XIVc.**
HPLC Rt = 2.84 min; [M+H]+ = 301. 1H NMR (400 MHz, DMSO-D6) δ ppm 2.61 (d, J=4.63 Hz, 3 H) 3.79 (s, 2 H) 7.10 (d, J=5.85 Hz, 1 H) 7.68 (d, J=9.15 Hz, 1 H) 8.01 (q, J=4.47 Hz, 1 H) 8.29 (dd, J=9.08, 2.26 Hz, 1 H) 9.02 (d, J=2.19

Hz, 1 H) 11.62 (s, 1 H) 12.80 (s, 1 H).

**Scheme 3**

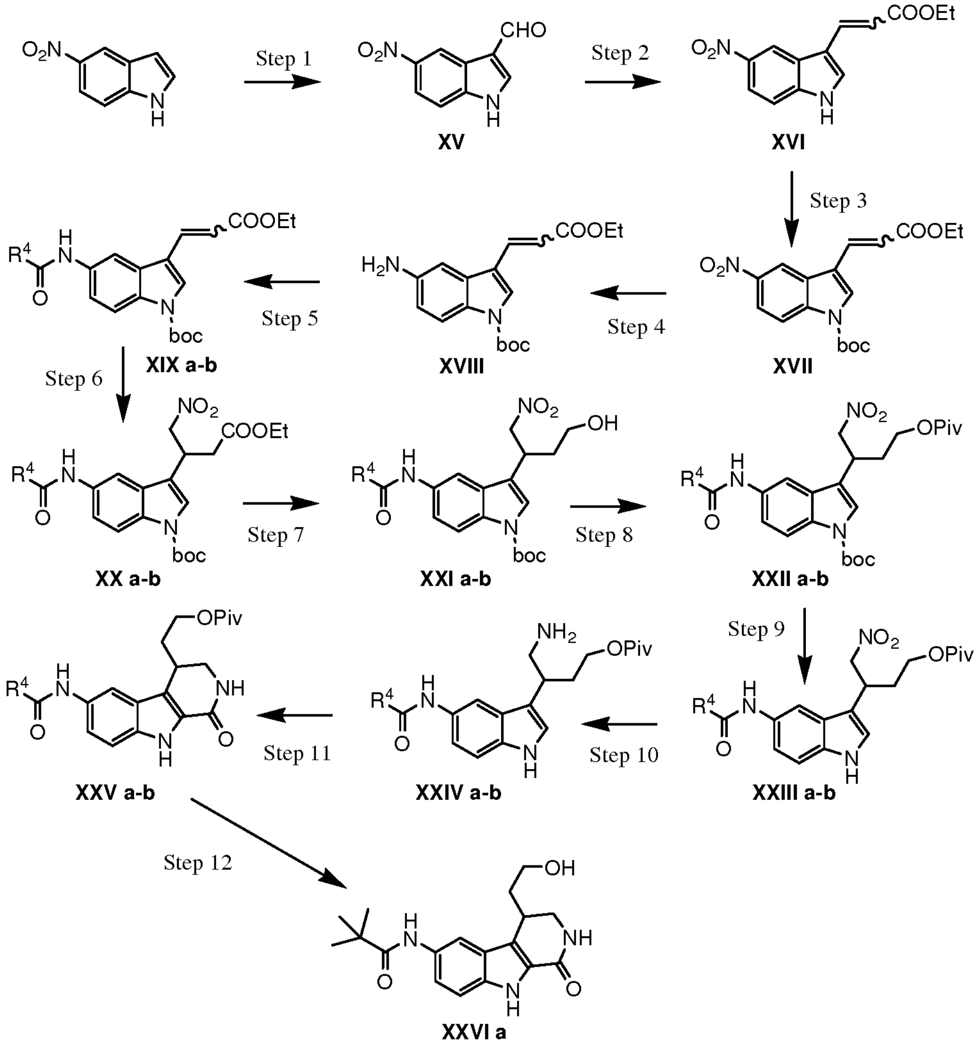


$R^4$ in scheme 4 carries the same meaning as in the compound of formula I described herein.

**Example 44**

**Compound XV: 5-Nitro-1H-indole-3-carbaldehyde**

$POCl_3$ (0.31 ml, 3.39 mmol, 1.1 eq) was slowly dropped, under argon atmosphere and vigorous stirring, into dry DMF (2 ml) and then cooled at -20 °C. After stirring for 30 min. at room temp., the solution was cooled at -20 °C, and 5-nitro indole (500 mg, 3.08 mol, 1 eq) in 0.5 ml of dry DMF was slowly added. The yellow solution was then stirred at room temperature for 1 h. A pale-yellow compound precipitated. The mixture was diluted with DMF, and then an equal volume of icy water was added. Aqueous NaOH (50% v/v) was then added until pH 9 was reached and the obtained red solution was refluxed, and then left overnight at 4°C.

The obtained crystals were filtered, washed with cool water and dried. Crystallization (EtOH-$H_2O$) afforded 574 mg of the title compound **XV**. Yield= 98 %.

1H-NMR (400 MHz, DMSO-d6) δ ppm 7.69 (d, J=9.0 Hz, 1 H) 8.12 (dd, J=2.3 Hz, 1 H) 8.54 (s, 1 H) 8.91 (d, 1 H) 9.99 (s, 1 H).

**Example 45**

**Compound XVI: Ethyl 3-(5-nitro-1H-indol-3-yl)acrylate**

A solution of $Ph_3PCHCOOEt$ (2.07 g, 5.97 mmol, 1.15 eq) in dry toluene (15 ml) was added, via cannula, to a suspension of 3-carboxaldehyde-5-nitroindole **XV** (987 mg, 5.19 mmol, 1 eq) in dry toluene (49 ml). The resulting suspension was heated at reflux under argon atmosphere until disappearance of starting material (about 2h) (TLC, hexane/acetone=6:4). The solvent was removed at reduced pressure and the crude was purified by flash chromatography (hexane/AcOEt=5:5) to afford the title compound **XVI** (1.34 g, mixture of isomers E/Z 9/1, quantitative yield). 1H-NMR (400 MHz, $CDCl_3$) $\delta$ ppm 1.38 (t, J=7 Hz, 3 H) 4.30 (q, J=7 Hz, 3 H) 6.53 (d, J=16.1 Hz, 1 H) 7.48 (d, J=9 Hz, 1 H) 7.64 (d, J=2.7 Hz, 1 H) 7.88 (d, J=16.1 Hz, 1 H) 8.20 (dd, J= 9, 1.9 Hz, 1 H) 8.86 (d, J=1.9 Hz, 1 H) 8.93 (broad, 1 H).

**Example 46**

**Compound XVII: Tert-butyl 3-(3-ethoxy-3-oxoprop-1-en-1-yl)-5-nitro-1H-indole-1-carboxylate**

To a suspension of **XVI,** mixture of isomers, (1.603 g, 6.16 mmol, 1 eq) in dry DCM (35 ml) under argon was added, via cannula, a solution of $Boc_2O$ (1.613 g, 7.39 mmol, 1.2 eq) and DMAP (752 mg, 6.16 mmol, 1 eq) in dry DCM (35 ml). After 10 minutes, the solution was extracted with aqueous citric acid 10% (also HCl 5% can be used) and the aqueous layer was extracted with DCM. The combined organic layer was dried with $Na_2SO_4$ and evaporated to afford the title compound **XVII** (2.21 g, 6.14 mmol, mixture of isomers, quantitative yield). The mixture was used without further purification.
E Isomer 1H-NMR (400 MHz, $CDCl_3$) $\delta$ ppm 1.38 (t, J=7 Hz, 3 H) 1.72 (s, 9 H) 4.30 (q, J=7 Hz, 3 H) 6.57 (dd, J=16.1, 0.4 Hz, 1 H) 7.80 (dd, J=16.1, 0.6 Hz, 1 H) 7.97 (s, 1 H) 8.27 (dd, J=9.1, 2.2 Hz, 1 H) 8.32 (d, J=9.1 Hz, 1 H) 8.73 (dd, J=2.2, 0.6 Hz, 1 H) Z Isomer 1H-NMR (400 MHz, CDCl3) $\delta$ ppm 1.33 (t, J=7 Hz, 3 H) 1.72 (s, 9 H) 4.27 (q, J=7 Hz, 3 H) 6.07 (d, J=12.5 Hz, 1 H) 7.08 (dd, J=12.5, 0.8 Hz, 1 H) 8.23 (dd, J=9.1, 2.2 Hz, 1 H) 8.30 (d, J=9.1 Hz, 1 H) 8.55 (d, J=2.2 Hz, 1 H) 9.02 (s, 1 H).

**Example 47**

**Compound XVIII: Tert-butyl 5-amino-3-(3-ethoxy-3-oxoprop-1-en-1-yl)-1H-indole-1-carboxylate**

Compound **XVII** (361 mg, 1.76 mmol, mixture of isomers, 1 eq) and $SnCl_2$ (2.67 g, 189 mmol, 8 eq) were dissolved in degassed EtOH (10 ml) and heated to reflux under argon atmosphere. As soon as TLC analysis (hexane/AcOEt=8:2) revealed the disappearance of the starting material (90 min), the solution was poured into ice and basified with aqueous NaOH (5%) (formation of a white suspension). The suspension was then extracted with AcOEt (3 times); the organic layer was washed with brine, dried with $Na_2SO_4$ and evaporated. (NOTE: it is important to eliminate tin salts, because they interfere in the reaction with PivCl). The title compound **XVIII** was obtained and used without further purification.

**Example 48**

**Compound XIXa: Tert-butyl 5-[(2,2-dimethylpropanoyl)amino]-3-(3-ethoxy-3-oxoprop-1-en-1-yl)-1H-indole-1-carboxylate**

Crude **XVIII** (580 mg, 1.76 mmol, 1 eq) was dissolved in dry DCM (10 ml) and cooled to 0°C. TEA (0.98 ml, 7.04 mmol, 4 eq) and PivCl (0.43 ml, 3.52 mmol, 2 eq) were added and the solution was stirred at RT for 10 min under argon atmosphere. EtOH was added in order to destroy the excess of PivCl and the solvent was evaporated. The crude was purified by flash chromatography (hexane/AcOEt=9:1 to 8:2) to afford the title compound **XIXa,** mixture of isomers (410 mg, 56% in the two steps).
E Isomer 1H-NMR (400 MHz, $CDCl_3$) $\delta$ ppm 1.36 (t, J=7 Hz, 3 H) 1.37 (s, 9 H) 1.67 (s, 9 H) 4.28 (q, J=7 Hz, 3 H) 6.49 (d, J=16.1 Hz, 1 H) 7.4-7.5 (m, 2 H) 7.78 (d, J=16.1 Hz, 1 H) 7.82 (broad, 1 H) 8.08 (broad, 1 H) 8.10 and 8.12 (broad, 1 H).

**Example 49**

**Compound XIXb: Tert-butyl 3-(3-ethoxy-3-oxoprop-1-en-1-yl)-5-[(phenylacetyl) amino]-1H-indole-1-carboxylate**

The same procedure was used for compound **XIXa,** using BzCl instead of PivCl. Compound **XIXb** was obtained

in 73% yield.

E Isomer 1H-NMR (400 MHz, $CDCl_3$) δ ppm 1.22 (t, J=7 Hz, 3 H) 1.66 (s, 9 H) 4.22 (q, J=7 Hz, 3H) 6.42 (d, 1 H) 7.2-8.2 (m, 11 H).

## Example 50

### Compound XXa: Tert-butyl 5-[(2,2-dimethylpropanoyl)amino]-3-[3-ethoxy-1-(nitromethyl)-3-oxopropyl]-1H-indole-1-carboxylate

Compound **XIXa** (1.25 g, 3.02 mmol, mixture of isomers, 1 eq) was dissolved with heating at 60°C in $CH_3NO_2$ (40 ml). DBU (0.54 ml, 3.63 mmol, 1.2 eq) was added and the resulting orange solution was heated at 60°C for 5 h. After cooling at RT, DCM was added, and the solution was washed with HCl (1% water solution). The aqueous layer was extracted with DCM (3 times) and the combined organic layer was dried with $Na_2SO_4$ and evaporated. The crude was dissolved in dry DCM (20 ml) under argon atmosphere, a solution of $Boc_2O$ (1.51 mmol, 0.5 eq) and DMAP (2.11 mmol, 0.7 eq) in dry DCM (5 ml) was added. After 10 minutes, the solution was extracted with aqueous citric acid (10%) and the aqueous layer was extracted with DCM. The organic layer was dried with $Na_2SO_4$, the solvent was evaporated and the crude was purified by flash chromatography (hexane/AcOEt=8:2) to afford the title compound **XXa** (1.05 g, yield 73%).

1H-NMR (400 MHz, $CDCl_3$) δ ppm 1.22 (t, J=7 Hz, 3H) 1.37 (s, 9 H) 1.67 (s, 9 H) 4.28 (q, J=7 Hz, 3 H) 6.49 (d, J=16.1 Hz, 1 H) 7.4-7.5 (m, 2 H) 7.78 (d, J=16.1 Hz, 1 H) 7.82 (broad, 1H) 8.08 (broad, 1H) 8.10 and 8.12 (broad, 1H).

## Example 51

### Compound XXb: Tert-butyl 3-[3-ethoxy-1-(nitromethyl)-3-oxopropyl]-5-[(phenylacetyl)amino]-1H-indole-1-carboxylate

Compound **XIXb** (1.25 g, 3.02 mmol, mixture of isomers, 1 eq) was dissolved with heating at 60°C in $CH_3NO_2$ (40 ml). DBU (0.54 ml, 3.63 mmol, 1.2 eq) was added and the resulting orange solution was heated at 60°C for 5 h. After cooling at RT, DCM was added, and the solution was washed with HCl (1% water solution). The aqueous layer was extracted with DCM (3 times) and the combined organic layer was dried with $Na_2SO_4$ and evaporated. The crude was dissolved in dry DCM (20 ml) under argon atmosphere, a solution of $Boc_2O$ (1.51 mmol, 0.5 eq) and DMAP (2.11 mmol, 0.7 eq) in dry DCM (5 ml) was added. After 10 minutes, the solution was extracted with aqueous citric acid (10%) and the aqueous layer was extracted with DCM. The organic layer was dried with $Na_2SO_4$, the solvent was evaporated and the crude was purified by flash chromatography (hexane/AcOEt=8:2) to afford the title compound **XXa** (1.05 g, yield 73%).

1H-NMR (400 MHz, $CDCl_3$) δ ppm 1.29 (t, J=7 Hz, 3 H) 1.66 (s, 9 H) 2.85-2.98 (m, 2 H) 4.05 (q, J=7 Hz, 3H) 4.20-4.28 (m, 1 H) 4.82 (d, 2 H) 7.3-8.1 (m, 10 H).

## Example 52

### Compound XXIa: Tert-butyl 5-[(2,2-dimethylpropanoyl)amino]-3-[3-hydroxy-1-(nitromethyl)propyl]-1H-indole-1-carboxylate

Compound **XXa** (220 mg, 0.463 mmol, 1 eq) was dissolved, under argon atmosphere, in dry $Et_2O$ (3 ml) and DCM (3 ml), $LiBH_4$ (40 mg, 1.85 mmol, 4 eq) was added in two portions and the solution was stirred at RT for 30 min. The solution was then cooled at 0°C and HCl 5% was added drop wise until an acidic pH was reached. The solution was warmed at RT, diluted with water and extracted with DCM. The combined organic layers were dried with $Na_2SO_4$ and the solvent was removed in vacuo. Purification by flash chromatography (hexane:AcOEt=5:5 to AcOEt) afforded title compound **XXIa** (180 mg, yield 90%).

1H-NMR (400 MHz, $CDCl_3$) δ ppm 1.38 (s, 9 H) 1.68 (s, 9 H) 1.96 (m, 2 H) 3.48-3.6 (m, 2 H) 3.92 (m, 1 H) 4.69 (m, 2 H) 7.2-8.0 (m , 5 H).

## Example 53

### Compound XXIb: Tert-butyl 3-[3-hydroxy-1-(nitromethyl)propyl]-5-[(phenylacetyl) amino]-1H-indole-1-carboxylate

Compound **XXb** (220 mg, 0.463 mmol, 1 eq) was dissolved, under argon atmosphere, in dry $Et_2O$ (3 ml) and

DCM (3 ml), $LiBH_4$ (40 mg, 1.85 mmol, 4 eq) was added in two portions and the solution was stirred at RT for 30 min. The solution was then cooled at 0°C and HCl 5% was added drop wise until an acidic pH was reached. The solution was warmed at RT, diluted with water and extracted with DCM. The combined organic layers were dried with $Na_2SO_4$ and the solvent was removed in vacuo. Purification by flash chromatography (hexane:AcOEt=5:5 to AcOEt) afforded title compound **XXIb.** Yield= 68%.
1H-NMR (400 MHz, $CDCl_3$) $\delta$ ppm 1.64 (s, 9 H) 1.97 (m, 2 H) 3.5-3.6 (m, 2 H) 3.95 (m, 1 H) 4.66 (m, 2 H) 7.3-7.5 (m, 5 H) 7.8-8.2 (m, 5 H).

**Example 54**

**Compound XXIIa: Tert-butyl 5-[(2,2-dimethylpropanoyl)amino]-3-{3-[(2,2-dimethylpropanoyl)oxy]-1-(nitromethyl)propyl}-1H-indole-1-carboxylate**

Compound **XXIa** (112 mg, 0.26 mmol, 1 eq) was dissolved in dry DCM (10 ml) under argon atmosphere; dry Py (0.42 ml, 5.17 mmol, 20 eq) and PivCl (0.32 ml, 2.58 mmol, 10 eq) were added and the solution was stirred till disappearance of starting material (about 2 h). The solution was diluted with DCM and washed with water. The aqueous layer was extracted with DCM and the combined organic layers were dried with $Na_2SO_4$ and evaporated. Flash chromatography (hexane/AcOEt=8:2) afforded XXIIa (113 mg, yield 84%).
1H-NMR (400 MHz, $CDCl_3$) $\delta$ ppm 1.19 (s, 9 H) 1.36 (s, 9 H) 1.66 (s, 9 H) 2.2 (m, 2 H) 3.9 (m, 2 H) 4.1 (m, 1 H) 4.70 (dd, J=12.3, 7.1 Hz, 1 H) 4.78 (dd, J=12.3, 7.7 Hz, 1 H) 7.17 (m, 1 H) 7.42 (broad, 2 H) 8.0-8.2 (broad, 2 H).

**Example 55**

**Compound XXIIb: Tert-butyl 3-{3-[(2,2-dimethylpropanoyl)oxy]-1-(nitromethyl)propyl}-5-[(phenylacetyl)amino]-1H-indole-1-carboxylate**

Compound **XXIb** (112 mg, 0.26 mmol, 1 eq) was dissolved in dry DCM (10 ml) under argon atmosphere; dry Py (0.42 ml, 5.17 mmol, 20 eq) and PivCl (0.32 ml, 2.58 mmol, 10 eq) were added and the solution was stirred till disappearance of starting material (about 2 h). The solution was diluted with DCM and washed with water. The aqueous layer was extracted with DCM and the combined organic layers were dried with $Na_2SO_4$ and evaporated. Flash chromatography (hexane/AcOEt=8:2) afforded **XXIIb.** Yield= 79%.
1H-NMR (400 MHz, $CDCl_3$) $\delta$ ppm 1.19 (s, 9 H) 1.68 (m, 9 H) 2.22 (m, 2 H) 3.92 (m, 2 H) 4.12 (m, 1 H) 4.77 (m, 2 H) 7.3- 8.2 (m, 10 H).

**Example 56**

**Compound XXIIIa: 3-{5-[(2,2-Dimethylpropanoyl)amino]-1H-indol-3-yl}-4-nitrobutyl pivalate**

A solution of **XXIIa** (50 mg, 0.096 mmol) in TFA (1 ml) and DCM (5 ml) was stirred for 90 min at rt. The solvent was evaporated under reduced pressure to give title compound **XXIIIa** (quantitative yield).

**Example 57**

**Compound XXIIIb: 4-Nitro-3-{5-[(phenylacetyl)amino]-1H-indol-3-yl}butyl pivalate**

Same procedure used for compound **XXIIIa,** using **XXIIb** instead of **XXIIa.** Yield= 95%.
1H-NMR (400 MHz, $CDCl_3$) $\delta$ ppm 1.23 (s, 9 H) 2.06-2.24 (m, 2 H) 3.83-3.94 (m, 2 H) 4.03-4.12 (m, 1 H) 4.61-4.74 (m, 2 H) 7.00-7.88 (m, 9 H) 8.09 (bs, 1 H), 8.57 (bs, 1 H).

**Example 58**

**Compound XXIVa: 4-Amino-3-{5-[(2,2-dimethylpropanoyl)amino]-1H-indol-3-yl}butyl pivalate**

To a solution of **XXIIIa** in dry EtOH under argon atmosphere, $NiCl_2 \cdot 6H_2O$ (1 eq.) was added. After cooling at 0°C, $NaBH_4$ (7 eq.) was cautiously added, to obtain a black suspension; which was warmed to RT and stirred for 1 h. The solvent was evaporated under reduced pressure. AcOEt and solid NaHCO3 were added, the solid residue was filtered on a celite bed and washed with AcOEt; the resulting clear solution was dried with $Na_2SO_4$ and evaporated to yield title compound **XXIVa** as a crude product. The crude product was submitted to the subsequent reaction.

## Example 59

### Compound XXIVb: 4-Amino-3-{5-[(phenylacetyl)amino]-1H-indol-3-yl}butyl pivalate

Compound **XXIIIb** (65 mg, 0.149 mmol, 1 eq) was dissolved under inert atmosphere in dry MeOH (1.5 ml), the solution was cooled to 0°C, $NiCl_2 \cdot 6H_2O$ (35 mg, 0.149 mmol, 1 eq) and $NaBH_4$ (39 mg, 1.04 mmol, 7 eq) were added. After 20 min the solvent was removed under reduced pressure and the crude was dissolved in AcOEt. Solid $NaHCO_3$ was added until the black solution turned almost colorless. The solid was filtered off and the solvent was removed under reduced pressure to yield the title compound **XXIVb** as a crude product. The crude product was directly used for the next step.

## Example 60

### Compound XXVa: 2-{6-[(2,2-Dimethylpropanoyl)amino]-1-oxo-2,3,4,9-tetrahydro-1H-beta-carbolin-4-yl}ethyl pivalate

Compound **XXIVa** (64 mg, 0.165 mmol, 1 eq) was dissolved, under inert atmosphere, in dry toluene (3 ml) and TEA (0.11 ml, 0.793 mmol, 4.8 eq) was added. A solution of triphosgene (39 mg, 0.13 mmol, 0.8 eq) in dry toluene (1 ml) was added and the reaction was stirred for 30 min. Then HBr 37% in AcOH (0.23 ml, 1.32 mmol, 8 eq) was added and the reaction heated to reflux for 30 min. The solution was cooled to r.t, and diluted with $H_2O$, and extracted with AcOEt. The organic layer was then washed with a saturated solution of $NaHCO_3$, $H_2O$ and then dried over $Na_2SO_4$. The solvent was removed under reduced pressure and flash chromatography (AcOEt) afforded 55 mg (80% yield) of the title compound **XXV** as a pure compound.

1H-NMR (400 MHz, $CDCl_3$) δ ppm 1.26 (s, 9 H) 1.34 (s, 9 H) 1.98-2.10 (m, 2 H) 3.29-3.31 (m, 1 H) 3.41-3.48 (m, 1 H) 3.78-3.82 (bdd, 1 H) 3.97-4.03 (m, 1 H) 4.13-4.20 (m, 1 H) 6.10 (bs, 1 H) 7.14 (dd, 1 H) 7.30-7.38 (m, 2 H) 7.82 (s, 1 H) 10.1 (s, 1 H)

13C-NMR (400 MHz, $CDCl_3$) δ 27.73 (C(CH3)3), 28.14 (C(CH3)3), 29.82 (C-4), 39.22 (C(CH3)3),39.85 (C(CH3)3), 47.08 (C-3), 62.79 (C-2'), 123.1, 125.3, 126.6, 131.3, 135.0, 163.0 (C=O), 176.7 (C=O), 178.6 (C=O).

## Example 61

### Compound XXVb: 2-{1-Oxo-6-[(phenylacetyl)amino]-2,3,4,9-tetrahydro-1H-(3-carbolin-4-yl}ethyl pivalate

Crude compound **XXIVb** (0.149 mmol) was dissolved under inert atmosphere in dry toluene (3 ml) and TEA (0.13 ml, 0.943 mmol) was added. A solution of triphosgene (47 mg, 0.158 mmol) in dry toluene (1 ml) was added and the reaction was stirred for 30 min. Then HBr (37% in AcOH, 1.96 mmol, 0.28 ml) was added and the reaction heated to reflux for 30 min. The solution was cooled to r.t, and diluted with $H_2O$, extracted with AcOEt, the organic layer was then washed with a saturated solution of $NaHCO_3$, $H_2O$ then dried over $Na_2SO_4$. The solvent was removed under reduced pressure and flash chromatography (AcOEt) afforded 41 mg (65% yield) of title compound **XXVb** as a pure compound.

1H-NMR (400 MHz, $CDCl_3$) δ ppm 1.20 (s, 9 H) 2.00-2.18 (m, 2 H) 3.24-3.30 (m, 1 H) 3.48-3.54 (m, 1 H), 3.88 (bdd, 1 H) 4.04-4.12 (m, 1 H) 4.16-4.24 (m, 1 H) 6.60 (bs, 1 H) 7.15-7.92 (m, 7 H) 8.01 (s, 1 H) 8.30 (s, 1 H) 10.7 (s, 1 H).

## Example 62

### Compound XXVIa: N-[4-(2-hydroxyethyl)-1-oxo-2,3,4,9-tetrahydro-1H-beta-carbolin-6-yl]-2,2-dimethylpropanamide

The compound **XXVa** (20 mg, 0.042 mmol, 1 eq) was suspended in $H_2O$/THF 1:4 (0.5 ml) and LiOH*$H_2O$ (0.256 mg, 0.0625 mmol, 1.5 eq) was added; the reaction mixture was stirred for 16h. Organic volatiles were removed and the aqueous phase was treated with 2N hydrochloric acid until pH=5-5.5. The aqueous phase was extracted twice with AcOEt, the combined organic layers were then washed with $H_2O$ and then dried over $Na_2SO_4$. The solvent was removed under reduced pressure to afford 10 mg of the title compound **XXVIa.**

HPLC Rt = 2.83 min; [M+H]+ = 330. 1H NMR (500 MHz, DMSO-D6) δ ppm 1.23 (s, 9H) 1.82 (dq, 2 H) 3.14 - 3.22 (m, 1 H) 3.47 - 3.55 (m, 2 H) 3.60 (dd, J=12.38, 4.91 Hz, 2 H) 4.57 (t, J=5.02 Hz, 1 H) 7.34 (d, J=40.98 Hz, 1 H) 7.48 (s, 1 H) 7.89 (s, 1 H) 8.98 - 9.25 (m, 1 H) 11.50 (s, 1 H).

From all of the above, it is understood to the skilled person artisan that when preparing the compounds of formula (I) according to the aforementioned processes, comprehensive of any variant thereof, optional functional groups

within the starting materials or the intermediates thereof, which could give rise to unwanted side reactions, need to be properly protected according to conventional techniques. Likewise, the conversion of these latter into the free deprotected compounds can be carried out according to known procedures.

By analogy, any compound of formula (I) which is susceptible of being salified can be easily converted into the corresponding acid addition salt, by working in the presence of any pharmaceutically acceptable acid, for instance selected among those previously reported.

As it will be readily appreciated, if the compounds of formula (I) prepared according to the process described above are obtained as a mixture of isomers, their separation into the single isomers of formula (I), according to conventional techniques, is also within the scope of the present invention.

Conventional techniques for racemate resolution include, for instance, partitioned crystallization of diastereoisomeric salt derivatives or preparative chiral HPLC.

## PHARMACOLOGY

The compounds of formula (I) are active as protein kinase inhibitors and are therefore useful, for instance, to restrict the unregulated proliferation of tumor cells.

In therapy, they can be used in the treatment of various tumors, such as those formerly reported, as well as in the treatment of other cell proliferative disorders such as psoriasis, vascular smooth cell proliferation associated with atherosclerosis and post-surgical stenosis and restenosis and in the treatment of Alzheimer's disease.

The compounds of the invention can be also active as inhibitors of other protein kinases such as, for instance, protein kinase C in different isoforms, Met, PAK-4, PAK-5, ZC-1, STLK-2, DDR-2, Aurora 1, Aurora 2, Bub-1, PLK, Chk1, Chk2, HER2, raf1, MEK1, MAPK, EGF-R, PDGF-R, FGF-R, IGF-R, VEGF-R, PI3K, weel kinase, Src, Abl, AKT, ILK, MK-2, IKK-2, Cdk in different isoforms, Nek, CK2, GSK3, SULU, PKA, PKC, PDK, RET, KIT, LCK, TRKA and thus be effective in the treatment of diseases associated with other protein kinases.

The inhibiting activity of putative Cdc7 inhibitors and the potency of selected compounds is determined through a method of assay based on the use of Dowex resin capture technology.

The assay consists of the transfer of radioactivity labeled phosphate moiety by the kinase to an acceptor substrate. The resulting 33P-labeled product is separated from unreacted tracer, transferred into a scintillation cocktail and light emitted is measured in a scintillation counter.

## Inhibition assay of Cdc7 activity

The inhibiting activity of putative Cdc7 inhibitors and the potency of selected compounds can be determined through a method of assay based on the use of Dowex resin capture technology.

The assay consists of the transfer of radioactivity labeled phosphate moiety by the kinase to an acceptor substrate. The resulting 33P-labeled product is separated from unreacted tracer, transferred into a scintillation cocktail and light emitted is measured in a scintillation counter.

The inhibition assay of Cdc7/Dbf4 activity is performed according to the following protocol:

> The MCM2 substrate is trans-phosphorylated by the Cdc7/Dbf4 complex in the presence of ATP traced with $\gamma^{33}$-ATP. The reaction is stopped by addition of Dowex resin in the presence of formic acid. Dowex resin particles capture unreacted $\gamma^{33}$-ATP and drag it to the bottom of the well while $^{33}$P phosphorylated MCM2 substrate remains in solution. The supernatant is collected, transferred into Optiplate plates and the extent of substrate phosphorylation is evaluated by $\beta$ counting.

The inhibition assay of Cdc7/Dbf4 activity was performed in 96 wells plate according to the following protocol:

To each well of the plate were added:

- 10 $\mu$l test compound (10 increasing concentrations in the nM to uM range to generate a dose-response curve). The solvent for test compounds contained 3% DMSO. (final concentration 1%).
- 10 $\mu$l substrate MCM2 (6 $\mu$M final concentration), a mixture of cold ATP (2 $\mu$M final concentration) and radioactive ATP (1/5000 molar ratio with cold ATP).
- 10 $\mu$l enzyme (Cdc7/Dbf4, 2 nM final concentration) that started the reaction. The buffer of the reaction consisted in 50 mM HEPES pH 7.9 containing 15 mM $MgCl_2$, 2 mM DTT, 3 uM $NaVO_3$, 2mM glycerophosphate and 0.2 mg/ml BSA.
- After incubation for 60 minutes at room temperature, the reaction was stopped by adding to each well 150 $\mu$l of Dowex resin in the presence of 150 mM formic acid. After another 60 min incubation, 50 $\mu$L of suspension were withdrawn and transferred into 96-well OPTIPLATEs containing 150 $\mu$l of MicroScint 40 (Packard); after 5-10 minutes

shaking the plates were read for 1 min in a Packard TOP-Count radioactivity reader.

$IC_{50}$ determination:

Inhibitors were tested at different concentrations ranging from 0.0005 to 10 μM. Experimental data were analyzed by the computer program Assay Explorer using the four parameter logistic equation:

$$y = bottom+(top-bottom)/(1+10^{\wedge}((\log IC_{50}-x)*slope))$$

where x is the logarithm of the inhibitor concentration, y is the response; y starts at bottom and goes to top with a sigmoid shape.

In addition the selected compounds have been characterized for specificity on Cdk2A, on a panel of ser/threo kinases strictly related to cell cycle (Cdk2/cyclin E, Cdk1/cyclin B1, Cdk4/Cyclin D1, Cdk5/p25), on IGF1-R, Aurora-2, AKT1.

**Inhibition assay of Cdk2/Cyclin A activity**

Kinase reaction:

1.5 μM histone H1 substrate, 25 μM ATP (0.2 μCi P33γ-ATP), 30 ng of baculovirus co-expressed Cdk2/Cyclin A, 10 μM inhibitor in a final volume of 100 μl buffer (TRIS HCl 10 mM pH 7.5, $MgCl_2$ 10 mM, 7.5 mM DTT) were added to each well of a 96 U bottom well plate. After 10 min at 37 °C incubation, reaction was stopped by 20 μl EDTA 120 mM.

Capture:

100 μl were transferred from each well from the kinase reaction to MultiScreen plate, to allow substrate binding to phosphocellulose filter. Plates were then washed 3 times with 150 μl/well PBS $Ca^{++}/Mg^{++}$ free and filtered by MultiScreen filtration system.

Detection:

Filters were allowed to dry at 37°C, then 100 μl/well scintillant were added and 33P labeled histone H1 was detected by radioactivity counting in the Top-Count instrument. Results: data were analyzed and expressed as % inhibition referred to total activity of enzyme (=100%).

All compounds showing inhibition ≥ 50 % were further analyzed in order to study and define potency ($IC_{50}$) as well as the kinetic-profile of inhibitor through Ki calculation. $IC_{50}$ determination: the protocol used was the same described above, where inhibitors were tested at different concentrations ranging from 0.0045 to 10 μM. Experimental data were analyzed by the computer program GraphPad Prizm using the four parameter logistic equation:

$$y = bottom+(top-bottom)/(1+10^{\wedge}((\log IC_{50}-x)*slope))$$

where x is the logarithm of the inhibitor concentration, y is the response; y starts at bottom and goes to top with a sigmoid shape.

Ki calculation:

Either the concentration of ATP and histone H1 substrate were varied: 4, 8, 12, 24, 48 μm for ATP (containing proportionally diluted $P^{33}$ μ-ATP) and 0.4, 0.8, 1.2, 2.4, 4.8 μM for histone were used in absence and presence of two different, properly chosen inhibitor concentrations.

Experimental data were analyzed by the computer program "SigmaPlot" for Ki determination, using a random bireactant system equation:

$$v = \frac{V_{max} \dfrac{(A)(B)}{aK_AK_B}}{1 + \dfrac{(A)}{K_A} + \dfrac{(B)}{K_B} + \dfrac{(A)(B)}{aK_AK_B}}$$

where A=ATP and B=histone H1.

**Inhibition assay of Cdk2/Cyclin E activity**

Kinase reaction:

1.5 μM histone H1 (Sigma # H-5505) substrate, 25 μM ATP (0.2 μCi $P^{33}\gamma$-ATP), 15 ng of baculovirus co-expressed cdk2/GST-Cyclin E, suitable concentrations of inhibitor in a final volume of 100 μl buffer (TRIS HCl 10 mM pH 7.5, $MgCl_2$ 10 mM, 7.5 mM DTT+ 0.2mg/ml BSA) were added to each well of a 96 U bottom well plate. After 10 min at 37 °C incubation, reaction was stopped by 20 μl EDTA 120 mM.

Capture:

100 μl were transferred from each well to MultiScreen plate, to allow substrate binding to phosphocellulose filter. Plates were then washed 3 times with 150 μl/well PBS $Ca^{++}/Mg^{++}$ free and filtered by MultiScreen filtration system.

Detection:

Filters were allowed to dry at 37°C, then 100 μl/well scintillant were added and $^{33}P$ labeled histone H1 was detected by radioactivity counting in the Top-Count instrument.

**Inhibition assay of Cdk1/Cyclin B1 activity**

Kinase reaction:

1.5 μM histone H1 (Sigma # H-5505) substrate, 25 μM ATP (0.2 μCi $P^{33}\mu$-ATP), 30 ng of baculovirus co-expressed Cdk1/Cyclin B1, suitable concentrations of inhibitor in a final volume of 100 μl buffer (TRIS HCl 10 mM pH 7.5, $MgCl_2$ 10 mM, 7.5 mM DTT+ 0.2mg/ml BSA) were added to each well of a 96 U bottom well plate. After 10 min at 37 °C incubation, reaction was stopped by 20 μl EDTA 120 mM.

Capture:

100 μl were transferred from each well to MultiScreen plate, to allow substrate binding to phosphocellulose filter. Plates were then washed 3 times with 150 μl/well PBS $Ca^{++}/Mg^{++}$ free and filtered by MultiScreen filtration system.

Detection:

Filters were allowed to dry at 37°C, then 100 μl/well scintillant were added and $^{33}P$ labeled histone H1 was detected by radioactivity counting in the Top-Count instrument.

**Inhibition assay Cdk4/Cyclin D1 activity**

Kinase reaction:

0.4 μM mouse GST-Rb (769-921) (# sc-4112 from Santa Cruz) substrate, 10 μM ATP (0.5 μCi $P^{33}\mu$-ATP), 100 ng of baculovirus expressed GST-Cdk4/GST-Cyclin D1, suitable concentrations of inhibitor in a final volume of 50 μl buffer (TRIS HCl 10 mM pH 7.5, $MgCl_2$ 10 mM, 7.5 mM DTT+ 0.2mg/ml BSA) were added to each well of a 96 U bottom well plate. After 40 min at 37 °C incubation, reaction was stopped by 20 μl EDTA 120 mM.

Capture:

60 μl were transferred from each well to MultiScreen plate, to allow substrate binding to phosphocellulose filter. Plates were then washed 3 times with 150 ul/well PBS $Ca^{++}/Mg^{++}$ free and filtered by MultiScreen filtration system.

Detection:

Filters were allowed to dry at 37°C, then 100 μl/well scintillant were added and $^{33}P$ labeled Rb fragment was detected by radioactivity counting in the Top-Count instrument.

**Inhibition assay of Cdk5/p25 activity**

The inhibition assay of Cdk5/p25 activity was performed according to the following protocol:

Kinase reaction:

1.0 μM biotinylated histone peptide substrate, 0.25 μCi P33g-ATP, 4 nM Cdk5/p25 complex, 0-100 μM inhibitor in a final volume of 100 μl buffer (Hepes 20 mM pH 7.5, $MgCl_2$ 15 mM, 1 mM DTT) were added to each well of a 96 U bottom well plate. After 20 min at 37 °C incubation, the reaction was stopped by the addition of 500 μg SPA beads in phosphate-buffered saline containing 0.1% Triton X-100, 50 μM ATP and 5 mM EDTA. The beads were allowed to settle, and the radioactivity incorporated in the 33P-labelled peptide was detected in a Top Count scintillation counter.

Results:

Data were analyzed and expressed as % Inhibition using the formula:

$$100X(1 - (Unknown - Bkgd)/(Enz.\ Control - Bkgd))$$

$IC_{50}$ values were calculated using a variation of the four parameter logistics equation:

$$Y = 100/[1 + 10\ \hat{}((LogEC50 - X)*Slope)]$$

Where X =log(μM) and Y = % Inhibition.

**Inhibition assay of IGF1-R activity**

The inhibition assay of IGF1-R activity was performed according to the following protocol:

Kinase reaction:

10 μM biotinylated MBP (Sigma cat. # M-1891) substrate, 0-20 μM inhibitor, 6 μM cold ATP, 2 nM $^{33}P$-ATP, and 22.5 ng IGF1-R (pre-incubated for 30 min at room temperature with cold 60 μM cold ATP) in a final volume of 30 μl buffer (50 mM HEPES pH 7.9, 3 mM $MnCl_2$, 1 mM DTT, 3 μM $NaVO_3$) were added to each well of a 96 U bottom well plate. After incubation for 35 min at room temperature, the reaction was stopped by addition of 100 μl PBS buffer containing 32 mM EDTA, 500 μM cold ATP, 0.1% Triton X100 and 10mg/ml streptavidin coated SPA beads. After 15 min incubation, 110 μl of suspension were withdrawn and transferred into 96-well OPTIPLATEs containing 100 μl of 5M CsCl. After 4 hours, the plates were read for 2 min in a Packard TOP-Count radioactivity reader.

Results: Experimental data were analyzed with the program GraphPad Prizm.

**Inhibition assay of Aurora-2 activity**

The inhibiting activity and the potency of selected compounds was determined through a method of assay based on the use of the streptavidin scintillation proximity assay beads (amershampharmacia biotech) run in a 96 well plates. At the end of the reaction, the biotinylated peptide substrate was captured with the beads and subsequently allowed to stratify using CsCl. When a radioactivity labeled phosphate moiety was transferred by the kinase to the beads-bound peptide, light emitted was measured in a scintillation counter.

The inhibition assay of Aurora-2 activity was performed in 96 wells plate according to the following protocol:

Kinase reaction:

8 μM biotinylated peptide (4 repeats of LRRWSLG), 10 μM ATP (0.5 uCi $P^{33}$g-ATP), 10 nM Aurora2, 10 μM inhibitor in a final volume of 60 μl buffer (HEPES 50 mM pH 7.0, $MgCl_2$ 10 mM, 1 mM DTT, 0.125 mg/ml BSA, 3μM orthovanadate) were added to each well of a 96 U bottom well plate. After 30 minutes at room temperature incubation, reaction was stopped and biotinylated peptide captured by adding 100 μl of bead suspension.

Stratification:

100 μl of CsCl 7.5 M were added to each well and let stand one hour before radioactivity was counted in the Top-Count instrument.

Results: data were analyzed and expressed as % inhibition referred to total activity of enzyme (=100%).

All compounds showing inhibition ≥ 60 % were further analyzed in order to study the potency of the inhibitor through $IC_{50}$ calculation. The protocol used was the same described above, except that serial dilution of the inhibitor was used. Experimental data were fitted by nonlinear regression using the following equation:

$$v = v_0 + \frac{(v_0 - v_b)}{1 + 10^{n(\log IC_{50} - \log[I])}}$$

with $v_b$ as the baseline velocity, v as the observed reaction velocity, $v_o$ as the velocity in the absence of inhibitors, and [I] as the inhibitor concentration.

**Inhibition assay of AKT-1 activity**

Test compounds are prepared as a 10 mM solution in 100% DMSO and distributed into 96 well plates:

i - for % inhibition studies, individual dilution plates at 1 mM, 100 μM and 10 μM are prepared in 100% DMSO, then diluted at a 3X concentration (30, 3 and 0.3 μM) in $ddH_2O$, 3% DMSO. A Multimek 96 (Beckman) is used for compound pipetting into test plates

ii - for $IC_{50}$ determination, compounds are diluted to 1 mM in 100% DMSO and plated into the first column of a microtiter plate (A1 to G1), 100 μl. Well H1 is left empty for the internal standard.

A Biomek 2000 (Beckman) is used for serial 1:3 dilutions in water, 3% DMSO, from column A1 to A10 and for all the 7 compounds in the plate. In a standard experiment, the highest concentration of all compounds is 30 μM that is diluted in the final test mixture at 10 μM.

Columns 11 and 12 are left available for total activity reference and background evaluation.

Assay scheme:

U bottom test plates are prepared either with 10 μl of the compound dilution (3X) per well, or 3% DMSO/water, and then placed onto a PlateTrak robotized station (Packard) together with one reservoir for the Enzyme mix (3X) and one for the ATP mix (3X). As the test starts, the robot (PlateTrak system, Perkin Elmer) takes 10 μl of ATP mix, makes an air gap inside the tips (10 μl) and aspirates 10 μl of Enzyme mix. The following dispensation into the plates allows the kinase reaction to start upon 3 cycles of mixing done by the robot itself.

At this point, the correct concentration is restored for all reagents. The robot incubates the plates for 60 minutes at room temperature, and then stops the reaction by pipetting 150 μl of Dowex resin into the reaction mix. It is essential to keep the resin well stirred before addition to the plates.

The resin is left another 60 minutes to settle down; the robot then takes 50 μl of supernatant from each well and dispenses them into an Optiplate (Packard) with 150 μl of Microscint 40 (Packard).

Counting:

Optiplates, covered by a plastic film to avoid radioactive spilling, are then mixed 10 minutes before counting in a Packard Top Count.

Operating as described above in the tests of inhibition activity on enzymes, or in an analogous way, the following data were obtained for 2-methyl-N-{4-[2-(methylamino)-2-oxoethyl]-1-oxo-2,9-dihydro-1H-beta-carbolin-6-yl}hexanamide (Compound XIIIh). The same compound was tested for cytotoxic activity on cells as described below.

Exponentially growing cells A2780 were seeded and incubated at 37°C in a humidified 5% $CO_2$ atmosphere. After 24 hours, scalar doses of test compound were added to the medium and cells were incubated for additional 72 hours.

At the end of treatment, cell proliferation was determined by a cellular ATP monitoring system which consists of a mixture of luciferase and luciferin, reconstituted in a lysis buffer; 100 μL/well of this solution were added to the cells. The plates were then shaken for 2 minutes in an orbital shaker and were read in a luminometer, with the signal linearly proportional to the cell number.

Concentration inhibiting cell proliferation by 50% ($IC_{50}$) in comparison to control cells was calculated and reported in the table below.

| ***Enzyme inhibition activity*** | |
|---|---|
| **$IC_{50}$ (μM)** | **Enzyme** |
| 0.423 | AUR1 |
| 0.051 | AUR2 |
| 1.700 | CDK2/CYCA |
| 2.516 | FGFR1 |
| 1.891 | GSK3beta |
| 1.762 | JAK3 |
| 1.304 | PDK1 |
| 0.866 | PKAalpha |
| 1.076 | RET |
| 1.919 | VEGFR2 |
| 0.594 | VEGFR3 |
| **Cytotoxic activity on cells** | |
| **$IC_{50}$ (μM)** | **Cell Type** |
| 3.607 | A2780 |

The compounds of the present invention can be administered either as single agents or, alternatively, in combination with known anticancer treatments such as radiation therapy or chemotherapy regimen in combination with cytostatic or cytotoxic agents, antibiotic-type agents, alkylating agents, antimetabolite agents, hormonal agents, immunological agents, interferon-type agents, cyclooxygenase inhibitors (e.g. COX-2 inhibitors), matrixmetalloprotease inhibitors, telomerase inhibitors, tyrosine kinase inhibitors, anti-growth factor receptor agents, anti-HER agents, anti-EGFR agents, anti-angiogenesis agents (e.g. angiogenesis inhibitors), farnesyl transferase inhibitors, ras-raf signal transduction pathway inhibitors, cell cycle inhibitors, other cdks inhibitors, tubulin binding agents, topoisomerase I inhibitors, topoisomerase II inhibitors, and the like.

If formulated as a fixed dose, such combination products employ the compounds of this invention within the dosage range described below and the other pharmaceutically active agent within the approved dosage range.

Compounds of formula (I) can be used sequentially with known anticancer agents when a combination formulation is inappropriate.

The compounds of formula (I) of the present invention, suitable for administration to a mammal, e.g., to humans, can be administered by the usual routes and the dosage level depends upon the age, weight, conditions of the patient and administration route.

For example, a suitable dosage adopted for oral administration of a compound of formula (I) can range from about 10 to about 500 mg per dose, from 1 to 5 times daily. The compounds of the invention can be administered in a

variety of dosage forms, e.g., orally, in the form tablets, capsules, sugar or film coated tablets, liquid solutions or suspensions; rectally in the form suppositories; parenterally, e.g., intramuscularly, or through intravenous and/or intrathecal and/or intraspinal injection or infusion.

Another aspect of the invention relates to pharmaceutical compositions comprising a compound of formula (I) or a pharmaceutically acceptable salt thereof in association with a pharmaceutically acceptable carrier. The pharmaceutically acceptable carrier can be an excipeint such as a diluent. The pharmaceutical compositions containing the compounds of the invention are usually prepared following conventional methods and are administered in a suitable pharmaceutical form.

For example, the solid oral forms can contain, together with the active compound, diluents, e.g., lactose, dextrose saccharose, sucrose, cellulose, corn starch or potato starch; lubricants, e.g., silica, talc, stearic acid, magnesium or calcium stearate, and/or polyethylene glycols; binding agents, e.g., starches, arabic gum, gelatine methylcellulose, carboxymethylcellulose or polyvinyl pyrrolidone; disintegrating agents, e.g., starch, alginic acid, alginates or sodium starch glycolate; effervescing mixtures; dyestuffs; sweeteners; wetting agents such as lecithin, polysorbates, laurylsulphates; and, in general, non-toxic and pharmacologically inactive substances used in pharmaceutical formulations. These pharmaceutical preparations can be manufactured in known manner, for example, by means of mixing, granulating, tabletting, sugar-coating, or film-coating processes.

The liquid dispersions for oral administration can be syrups, emulsions or suspensions. As an example, the syrups can contain, as carrier, saccharose or saccharose with glycerine and/or mannitol and sorbitol. The suspensions and the emulsions can contain, as examples of carriers, natural gum, agar, sodium alginate, pectin, methylcellulose, carboxymethylcellulose, or polyvinyl alcohol.

The suspension or solutions for intramuscular injections can contain, together with the active compound, a pharmaceutically acceptable carrier, e.g., sterile water, olive oil, ethyl oleate, glycols, e.g., propylene glycol and, if desired, a suitable amount of lidocaine hydrochloride.

The solutions for intravenous injections or infusions can contain, as a carrier, sterile water. Preferably, they can be in the form of sterile, aqueous, isotonic, saline solutions or they can contain propylene glycol as a carrier.

The suppositories can contain, together with the active compound, a pharmaceutically acceptable carrier, e.g., cocoa butter, polyethylene glycol, a polyoxyethylene sorbitan fatty acid ester surfactant or lecithin.

With the aim to better illustrate the present invention, without posing any limitation to it, the following examples are now given.

## General methods

Flash Chromatography was performed on silica gel (Merck grade 9395, 60A). HPLC was performed on Waters X Terra RP 18 (4,6 x 50 mm, 3.5 μm) column using a Waters 2790 HPLC system equipped with a 996 Waters PDA detector and Micromass mod. ZQ single quadrupole mass spectrometer, equipped with an electrospray (ESI) ion source. Mobile phase A was ammonium acetate 5 mM buffer (pH 5.5 with acetic acid/acetonitrile 95:5), and Mobile phase B was $H_2O$/acetonitrile (5:95). Gradient from 10 to 90% B in 8 minutes, hold 90% B 2 minutes. UV detection at 220 nm and 254 nm. Flow rate 1 ml/min. Injection volume 10 μl. Full scan, mass range from 100 to 800 amu. Capillary voltage was 2.5 KV; source temp. was 120°C; cone was 10 V. Retention times (HPLC r.t.) are given in minutes at 220 nm or at 254 nm. Mass are given as m/z ratio. When necessary, compounds have been purified by preparative HPLC on a Waters Symmetry C18 (19 x 50 mm, 5 μm) column using a Waters preparative HPLC 600 equipped with a 996 Waters PDA detector and a Micromass mod. ZMD single quadrupole mass spectrometer, electron spray ionization, positive mode. Mobile phase A was water 0.01% TFA, and Mobile phase B was acetonitrile. Gradient from 10 to 90% B in 8 min, hold 90% B 2 min. Flow rate 20 ml/min.

1H-NMR spectrometry was performed on a Mercury VX 400 operating at 400.45 MHz equipped with a 5 mm double resonance probe [1H (15N-31P) ID_PFG Varian].

The compounds of formula (I), having an asymmetric carbon atom and obtained as racemic mixture, were resolved by HPLC separation on chiral columns. In particular, for example, preparative columns CHIRALPACK® AD, CHIRALPACK® AS, CHIRALCELL® OJ can be used.

## Claims

1. A compound represented by formula (I)

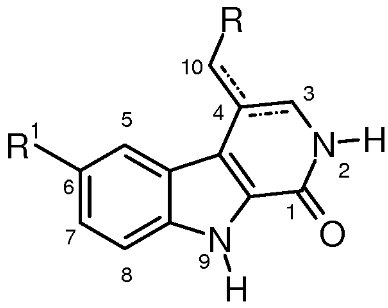

**(I)**

wherein

the lines between carbon 3 and carbon 4; and between carbon 4 and carbon 10, represent a single or double bond with the provisos that the bonds between C3-C4 and C4-C10 cannot be both double bonds;
**R** is selected from the group consisting of $CH_2OH$, $-CH_2OCOR^2$, $-CONR^2R^3$, and $-COOR^2$;
$\mathbf{R^1}$ is selected from the group consisting of $-NO_2$, $-NHR^4$, $-NHCOR^4$, $-NHCONR^4R^5$, $-NHSO_2R^4$ and $-NHCOOR^4$;
wherein $R^2$, $R^3$, $R^4$, and $R^5$, which can be the same or different, are each independently selected from the group consisting of hydrogen, $(C_1-C_6)$alkyl, $(C_2-C_6)$alkenyl, $(C_2-C_6)$alkynyl, $(C_3-C_6)$cycloalkyl, cycloalkyl$(C_1-C_6)$alkyl, carbocyclic, $(C_1-C_6)$alkyl heterocycle, aryl, aryl$(C_1-C_6)$alkyl, $(C_1-C_6)$alkylaryl, heterocycle, and heterocycle$(C_1-C_6)$alkyl, or, either $R^2$ and $R^3$ as well as $R^4$ and $R^5$, taken together with the nitrogen atom to which they are bonded, can each form an optionally substituted 4 to 7 membered heterocycle, containing one to three heteroatoms or heteroatomic groups selected from S, O, N or NH;
or a pharmaceutically acceptable salt or solvate thereof.

**2.** A compound according to claim 1 selected from the group consisting of:

methyl (6-nitro-1-oxo-2,9-dihydro-1H-beta-carbolin-4-yl)acetate;
(6-nitro-1-oxo-2,9-dihydro-1H-beta-carbolin-4-yl)acetic acid;
(6-nitro-1-oxo-2,9-dihydro-1H-beta-carbolin-4-yl)acetic ester resin bound;
N-{4-[2-(isopropylamino)-2-oxoethyl]-1-oxo-2,9-dihydro-1H-beta-carbolin-6-yl}nicotinamide;
N-isopropyl-2-{1-oxo-6-[(phenylacetyl)amino]-2,9-dihydro-1H-beta-carbolin-4-yl}acetamide;
N-{4-[2-(isopropylamino)-2-oxoethyl]-1-oxo-2,9-dihydro-1H-beta-carbolin-6-yl}-2-methylpropanamide;
N-{4-[2-(methylamino)-2-oxoethyl]-1-oxo-2,9-dihydro-1H-beta-carbolin-6-yl}-2-{4-[(methylsulfonyl)amino]phenyl} acetamide;
2-(3,4-dimethoxyphenyl)-N-{4-[2-(methylamino)-2-oxoethyl]-1-oxo-2,9-dihydro-1H-beta-carbolin-6-yl}acetamide
2,2-dimethyl-N-{4-[2-(methylamino)-2-oxoethyl]-1-oxo-2,9-dihydro-1H-beta-carbolin-6-yl}propanamide;
2-methyl-N-{4-[2-(methylamino)-2-oxoethyl]-1-oxo-2,9-dihydro-1H-beta-carbolin-6-yl}hexanamide;
1-methyl-N-{4-[2-(methylamino)-2-oxoethyl]-1-oxo-2,9-dihydro-1H-beta-carbolin-6-yl}-1H-pyrrole-2-carboxamide;
2-(2-methoxyphenyl)-N-{4-[2-(methylamino)-2-oxoethyl]-1-oxo-2,9-dihydro-1H-beta-carbolin-6-yl}acetamide;
N-{4-[2-(methylamino)-2-oxoethyl]-1-oxo-2,9-dihydro-1H-beta-carbolin-6-yl}tetrahydrofuran-2-carboxamide;
N-{4-[2-(methylamino)-2-oxoethyl]-1-oxo-2,9-dihydro-1H-beta-carbolin-6-yl}-2-(3-methylphenyl) acetamide;
5-methyl-N-{4-[2-(methylamino)-2-oxoethyl]-1-oxo-2,9-dihydro-1H-beta-carbolin-6-yl}pyrazine-2-carboxamide;
5-bromo-N-{4-[2-(methylamino)-2-oxoethyl]-1-oxo-2,9-dihydro-1H-beta-carbolin-6-yl}-2-furamide;
N-methyl-2-(1-oxo-6-{[(pyridin-4-ylthio)acetyl]amino}-2,9-dihydro-1H-beta-carbolin-4-yl)acetamide;
N-benzyl-2-(6-nitro-1-oxo-2,9-dihydro-1H-beta-carbolin-4-yl)acetamide;
N-isopropyl-2-(6-nitro-1-oxo-2,9-dihydro-1H-beta-carbolin-4-yl)acetamide;
N-methyl-2-(6-nitro-1-oxo-2,9-dihydro-1H-beta-carbolin-4-yl)acetamide; and
N-[4-(2-hydroxyethyl)-1-oxo-2,3,4,9-tetrahydro-1H-beta-carbolin-6-yl]-2,2-dimethylpropanamide.

**3.** A compound according to claim 1, wherein R is $-CONR^2R^3$; and $R^1$ is $-NHR^4$ or $-NHCOR^4$.

**4.** A compound according to claim 1, wherein R is $-CONR^2R^3$, wherein $R^2$ and $R^3$ are each independently hydrogen

or alkyl; and $R^1$ is $-NHCOR^4$.

**5.** A pharmaceutical composition comprising an amount of the compound of claim 1, or a pharmaceutically acceptable salt or solvate thereof, and a pharmaceutically acceptable carrier.

**6.** A compound according to claim 1 or a pharmaceutical composition according to claim 5 for a therapeutic use.

**7.** A compound or a pharmaceutical composition according to claim 6 wherein said therapeutic use is for treating a cell proliferative disorder or condition in a mammal.

**8.** A compound or a pharmaceutical composition according to claim 7, wherein said disorder or condition is caused by or is associated with an altered Cdk2 or Cdc7 kinase activity.

**9.** A compound or a pharmaceutical composition according to claim 7 wherein said disorder or condition is selected from the group consisting of carcinoma, squamous cell carcinoma, hematopoietic tumors of myeloid or lymphoid lineage, tumors of mesenchymal origin, tumors of the central and peripheral nervous system, melanoma, seminoma, teratocarcinoma, osteosarcoma, xeroderma pigmentosum, keratoxanthoma, thyroid follicular cancer, and Kaposi's sarcoma

**10.** A compound or a pharmaceutical composition according to claim 7 wherein said disorder or condition is selected from the group consisting of benign prostate hyperplasia, familial adenomatosis, polyposis, neuro-fibromatosis, psoriasis, vascular smooth cell proliferation associated with atherosclerosis, pulmonary fibrosis, arthritis glomerulonephritis, post-surgical stenosis and restenosis.

**11.** A compound or a pharmaceutical composition according to claim 7 wherein said disorder or condition is selected from the group consisting of carcinoma, squamous cell carcinoma, hematopoietic tumors of myeloid or lymphoid lineage, tumors of mesenchymal origin, tumors of the central and peripheral nervous system, melanoma, seminoma, teratocarcinoma, osteosarcoma, xeroderma pigmentosum, keratoxanthoma, thyroid follicular cancer, and Kaposi's sarcoma

**12.** A compound or a pharmaceutical composition according to claim 7 wherein said disorder or condition is selected from the group consisting of squamous cell carcinoma, leukemia, acute lymphocitic leukemia, acute lymphoblastic leukemia, B-cell lymphoma, T-cell-lymphoma, Hodgkin's lymphoma, non-Hodgkin's lymphoma, hairy cell lymphoma and Burkitt's lymphoma, acute and chronic myelogenous leukemias, myelodysplastic syndrome, promyelocytic leukemia, fibrosarcoma, rhabdomyosarcoma, astrocytoma, neuroblastoma, glioma, schwannomas, melanoma, seminoma, teratocarcinoma, osteosarcoma, xeroderma pigmentosum, keratoxanthoma, thyroid follicular cancer , Kaposi's sarcoma and carcinoma of the bladder, breast, colon, kidney, liver, lung, esophagus, gall-bladder, ovary, pancreas, stomach, cervix, thyroid, prostate, or skin.

**13.** A product or kit comprising a compound of formula (I) or a pharmaceutically acceptable salt thereof, as defined in claim 1, or pharmaceutical compositions thereof as defined in claim 5, and one or more chemotherapeutic agent, as a combined preparation for simultaneous, separate or sequential use in anticancer therapy.

**14.** Use of a compound of formula (I) or a pharmaceutically acceptable salt thereof, as defined in claim 1, in the manufacture of a medicament with antitumor activity.

**Patentansprüche**

**1.** Verbindung, dargestellt durch Formel (I),

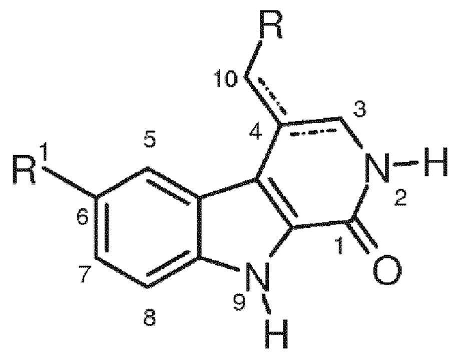

(I)

wobei

die Linien zwischen Kohlenstoff 3 und Kohlenstoff 4 sowie zwischen Kohlenstoff 4 und Kohlenstoff 10 eine Einzel- oder Doppelbindung darstellen, mit den Maßgaben, dass die Bindungen zwischen C3-C4 und C4-C10 nicht beide Doppelbindungen sein können;
**R** aus der Gruppe, bestehend aus $CH_2OH$, $-CH_2OCOR^2$, $-CONR^2R^3$ und $-COOR^2$, ausgewählt ist;
**$R^1$** aus der Gruppe, bestehend aus $-NO_2$, $-NHR^4$, $-NHCOR^4$, $-NHCONR^4R^5$, $-NHSO_2R^4$ und $-NHCOOR^4$, ausgewählt ist;
wobei $R^2$, $R^3$, $R^4$ und $R^5$, die gleich oder unterschiedlich sein können, jeweils unabhängig aus der Gruppe ausgewählt sind, die aus Wasserstoff, $(C_1-C_6)$ -Alkyl, $(C_2-C_6)$ -Alkenyl, $(C_2-C_6)$ -Alkinyl, $(C_3-C_6)$-Cycloalkyl, Cycloalkyl-$(C_1-C_6)$-alkyl, Carbocyclus, $(C_1-C_6)$-Alkyl-heterocyclus, Aryl, Aryl-$(C_1-C_6)$-alkyl, $(C_1-C_6)$-Alkylaryl, Heterocyclus und Heterocyclus-$(C_1-C_6)$-alkyl besteht, oder $R^2$ und $R^3$ sowie $R^4$ und $R^5$ zusammengefasst mit dem Stickstoffatom, an das sie gebunden sind, jeweils einen wahlweise substituierten 4- bis 7-gliedrigen Heterocyclus bilden können, der ein bis drei Heteroatome oder heteroatomare Gruppen, ausgewählt aus S, O, N und NH, enthält;
oder ein pharmazeutisch annehmbares Salz oder Solvat davon.

**2.** Verbindung nach Anspruch 1, ausgewählt aus der Gruppe, bestehend aus:

Methyl(6-nitro-1-oxo-2,9-dihydro-1H-beta-carbolin-4-yl)acetat;
(6-Nitro-1-oxo-2,9-dihydro-1H-beta-carbolin-4-yl)essigsäure;
(6-Nitro-1-oxo-2,9-dihydro-1H-beta-carbolin-4-yl)essigsäureester, an Harz gebunden;
N-{4-[2-(Isopropylamino)-2-oxoethyl]-1-oxo-2,9-dihydro-1H-beta-carbolin-6-yl}nicotinamid;
N-Isopropyl-2-{1-oxo-6-[(phenylacetyl)amino]-2,9-dihydro-1H-beta-carbolin-4-yl}acetamid;
N-{4-[2-(Isopropylamino)-2-oxoethyl]-1-oxo-2,9-dihydro-1H-beta-carbolin-6-yl}-2-methylpropanamid;
N-{4-[2-(Methylamino)-2-oxoethyl]-1-oxo-2,9-dihydro-1H-beta-carbolin-6-yl}-2-{4-[(methylsulfonyl)amino]phenyl}acetamid;
2-(3,4-Dimethoxyphenyl)-N-{4-[2-(methylamino)-2-oxoethyl]-1-oxo-2,9-dihydro-1H-beta-carbolin-6-yl} acetamid;
2,2-Dimethyl-N-{4-[2-(methylamino)-2-oxoethyl]-1-oxo-2,9-dihydro-1H-beta-carbolin-6-yl}propanamid;
2-Methyl-N-{4-[2-(methylamino)-2-oxoethyl]-1-oxo-2,9-dihydro-1H-beta-carbolin-6-yl}hexanamid;
1-Methyl-N-{4-[2-(methylamino)-2-oxoethyl]-1-oxo-2,9-dihydro-1H-beta-carbolin-6-yl}-1H-pyrrol-2-carboxamid;
2-(2-Methoxyphenyl)-N-{4-[2-(methylamino)-2-oxoethyl]-1-oxo-2,9-dihydro-1H-beta-carbolin-6-yl}acetamid;
N-{4-[2-(Methylamino)-2-oxoethyl]-1-oxo-2,9-dihydro-1H-beta-carbolin-6-yl}tetrahydrofuran-2-carboxamid;
N-{4-[2-(Methylamino)-2-oxoethyl]-1-oxo-2,9-dihydro-1H-beta-carbolin-6-yl}-2-(3-methylphenyl)acetamid;
5-Methyl-N-{4-[2-(methylamino)-2-oxoethyl]-1-oxo-2,9-dihydro-1H-beta-carbolin-6-yl}pyrazin-2-carboxamid;
5-Brom-N-{4-[2-(methylamino)-2-oxoethyl]-1-oxo-2,9-dihydro-1H-beta-carbolin-6-yl}-2-furamid;
N-Methyl-2-(1-oxo-6-{[(pyridin-4-ylthio)acetyl]amino}-2,9-dihydro-1H-beta-carbolin-4-yl)acetamid;
N-Benzyl-2-(6-nitro-1-oxo-2,9-dihydro-1H-beta-carbolin-4-yl)acetamid;
N-Isopropyl-2-(6-nitro-1-oxo-2,9-dihydro-1H-beta-carbolin-4-yl)acetamid;
N-Methyl-2-(6-nitro-1-oxo-2,9-dihydro-1H-beta-carbolin-4-yl)acetamid; und
N-[4-(2-Hydroxyethyl)-1-oxo-2,3,4,9-tetrahydro-1H-beta-carbolin-6-yl]-2,2-dimethylpropanamid.

3. Verbindung nach Anspruch 1, wobei R $-CONR^2R^3$ ist; und $R^1$ $-NHR^4$ oder $-NHCOR^4$ ist.

4. Verbindung nach Anspruch 1, wobei R $-CONR^2R^3$ ist, wobei $R^2$ und $R^3$ jeweils unabhängig Wasserstoff oder Alkyl sind; und $R^1$ $-NHCOR^4$ ist.

5. Pharmazeutische Zusammensetzung, umfassend eine Menge der Verbindung nach Anspruch 1 oder eines pharmazeutisch annehmbaren Salzes oder Solvats davon und einen pharmazeutisch annehmbaren Träger.

6. Verbindung nach Anspruch 1 oder pharmazeutische Zusammensetzung nach Anspruch 5 zu einer therapeutischen Benutzung.

7. Verbindung oder pharmazeutische Zusammensetzung nach Anspruch 6, wobei die therapeutische Benutzung zum Behandeln einer zellproliferativen Störung oder eines zellproliferativen Zustandes bei einem Säuger ist.

8. Verbindung oder pharmazeutische Zusammensetzung nach Anspruch 7, wobei die Störung oder der Zustand durch eine veränderte Cdk2- oder Cdc7-Kinase-Aktivität verursacht oder damit verbunden ist.

9. Verbindung oder pharmazeutische Zusammensetzung nach Anspruch 7, wobei die Störung oder der Zustand aus der Gruppe ausgewählt ist, die aus Karzinom, Plattenepithelkarzinom, hämatopoetischen Tumoren der myeloischen oder lymphoiden Reihe, Tumoren mesenchymalen Ursprungs, Tumoren des zentralen und peripheren Nervensystems, Melanom, Seminom, Teratokarzinom, Osteosarkom, Xeroderma pigmentosum, Keratoxanthom, follikulärem Schilddrüsenkrebs und Kaposi-Sarkom besteht.

10. Verbindung oder pharmazeutische Zusammensetzung nach Anspruch 7, wobei die Störung oder der Zustand aus der Gruppe ausgewählt ist, die aus gutartiger Prostatahyperplasie, familiärer Adenomatose, Polypose, Neurofibromatose, Psoriasis, Proliferation vaskulärer glatter Zellen im Zusammenhang mit Atherosklerose, Lungenfibrose, Arthritis, Glomerulonephritis, postoperativer Stenose und Restenose besteht.

11. Verbindung oder pharmazeutische Zusammensetzung nach Anspruch 7, wobei die Störung oder der Zustand aus der Gruppe ausgewählt ist, die aus Karzinom, Plattenepithelkarzinom, hämatopoetischen Tumoren der myeloischen oder lymphoiden Reihe, Tumoren mesenchymalen Ursprungs, Tumoren des zentralen und peripheren Nervensystems, Melanom, Seminom, Teratokarzinom, Osteosarkom, Xeroderma pigmentosum, Keratoxanthom, follikulärem Schilddrüsenkrebs und Kaposi-Sarkom besteht.

12. Verbindung oder pharmazeutische Zusammensetzung nach Anspruch 7, wobei die Störung oder der Zustand aus der Gruppe ausgewählt ist, die aus Plattenepithelkarzinom, Leukämie, akuter lymphozytischer Leukämie, akuter lymphoblastischer Leukämie, B-Zell-Lymphom, T-Zell-Lymphom, Hodgkin-Lymphom, Non-Hodgkin-Lymphom, Haarzellenlymphom und Burkitt-Tumor, akuten und chronischen myeloischen Leukämien, myelodysplastischem Syndrom, Promyelozytenleukämie, Fibrosarkom, Rhabdomyosarkom, Astrozytom, Neuroblastom, Gliom, Schwannomen, Melanom, Seminom, Teratokarzinom, Osteosarkom, Xeroderma pigmentosum, Keratoxanthom, follikulärem Schilddrüsenkrebs, Kaposi-Sarkom und Karzinom der Blase, Brust, des Dickdarms, der Niere, Leber, Lunge, Speiseröhre, Gallenblase, des Eierstocks, der Bauchspeicheldrüse, des Magens, Gebärmutterhalses, der Schilddrüse, Prostata oder Haut besteht.

13. Produkt oder Kit, umfassend eine Verbindung mit der Formel (I) oder ein pharmazeutisch annehmbares Salz davon, wie in Anspruch 1 definiert, oder pharmazeutische Zusammensetzungen davon, wie in Anspruch 5 definiert, und ein oder mehrere chemotherapeutische Mittel als ein Kombinationspräparat zum gleichzeitigen, separaten oder nacheinander erfolgenden Benutzung in einer Krebstherapie.

14. Benutzung einer Verbindung mit der Formel (I) oder eines pharmazeutisch annehmbaren Salzes davon, wie in Anspruch 1 definiert, zur Herstellung eines Arzneimittels mit Antitumoraktivität.

## Revendications

1. Composé représenté par la formule (I)

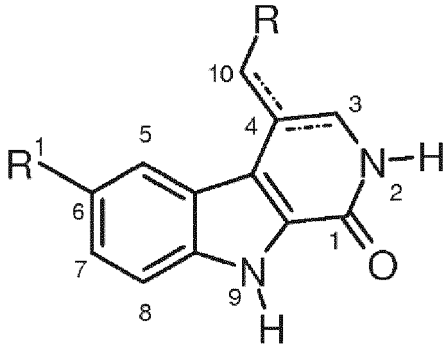

(I)

dans lequel

les lignes entre le carbone 3 et le carbone 4 ; et entre le carbone 4 et le carbone 10, représentent une liaison simple ou double à condition que les liaisons entre les $C_3$-$C_4$ et $C_4$-$C_10$ ne puissent pas être les deux des liaisons doubles ;
**R** est sélectionné dans le groupe constitué de $CH_2OH$,-$CH_2OCOR^2$, -$CONR^2R^3$, et -$COOR^2$ ;
**R[1]** est sélectionné dans le groupe constitué de -$NO_2$,-$NHR^4$, -$NHCOR^4$, -$NHCONR^4R^5$, -$NHSO_2R^4$ et -$NHCOOR^4$ ;
dans lequel $R^2$, $R^3$, $R^4$, et $R^5$, qui peuvent être identiques ou différents, sont chacun indépendamment sélectionnés dans le groupe constitué d'un atome d'hydrogène, des groupes alkyle en $C_1$-$C_6$, alcényle en $C_2$-$C_6$, alcynyle en $C_2$-$C_6$, cycloalkyle en $C_3$-$C_6$, cycloalkyl(alkyle en $C_1$-$C_6$), carbocyclique, -(alkyle en $C_1$-$C_6$) hétérocycle, aryle, -aryl (alkyle en $C_1$-$C_6$), alkylaryle en $C_1$-$C_6$, hétérocycle, et hétérocycle (alkyle en $C_1$-$C_6$), ou, soit $R^2$ et $R^3$ soit $R^4$ et $R^5$, pris conjointement à l'atome d'azote auquel ils sont liés, peuvent chacun former un hétérocycle à 4 à 7 chaînons facultativement substitué, contenant un à trois hétéroatomes ou groupes hétéroatomiques sélectionnés parmi S, O, N ou NH ;
ou un sel ou un solvate pharmaceutiquement acceptable de celui-ci.

2. Composé selon la revendication 1 sélectionné dans le groupe constitué des :

(6-nitro-1-oxo-2,9-dihydro-1H-bêta-carbolin-4-yl)acétate de méthyle ;
acide (6-nitro-1-oxo-2,9-dihydro-1H-bêta-carbolin-4-yl)acétique ;
ester (6-nitro-1-oxo-2,9-dihydro-1H-bêta-carbolin-4-yl)-acétique lié à une résine ;
N-{4-[2-(isopropylamino)-2-oxoéthyl]-1-oxo-2,9-dihydro-1H-bêta-carbolin-6-yl}nicotinamide ;
N-isopropyl-2-{1-oxo-6-[(phénylacétyl)amino]-2,9-dihydro-1H-bêta-carbolin-4-yl}acétamide ;
N-{4-[2-(isopropylamino)-2-oxoéthyl]-1-oxo-2,9-dihydro-1H-bêta-carbolin-6-yl}-2-méthylpropanamide ;
N-{4-[2-(méthylamino)-2-oxoéthyl]-1-oxo-2,9-dihydro-1H-bêta-carbolin-6-yl}-2-{4-[(méthylsulfonyl)amino]phényl}acétamide ;
2-(3,4-diméthoxyphényl)-N-{4-[2-(méthylamino)-2-oxoéthyl]-1-oxo-2,9-dihydro-1H-bêta-carbolin-6-yl}acétamide
2,2-diméthyl-N-{4-[2-(méthylamino)-2-oxoéthyl]-1-oxo-2,9-dihydro-1H-bêta-carbolin-6-yl}propanamide ;
2-méthyl-N-{4-[2-(méthylamino)-2-oxoéthyl]-1-oxo-2,9-dihydro-1H-bêta-carbolin-6-yl} hexanamide ;
1-méthyl-N-{4-[2-(méthylamino)-2-oxoéthyl]-1-oxo-2,9-dihydro-1H-bêta-carbolin-6-yl}-1H-pyrrole-2-carboxamide ;
2-(2-méthoxyphényl)-N-{4-[2-(méthylamino)-2-oxoéthyl]-1-oxo-2,9-dihydro-1H-bêta-carbolin-6-yl}acétamide ;
N-{4-[2-(méthylamino)-2-oxoéthyl]-1-oxo-2,9-dihydro-1H-bêta-carbolin-6-yl}tétrahydrofuran-2-carboxamide ;
N-{4-[2-(méthylamino)-2-oxoéthyl]-1-oxo-2,9-dihydro-1H-bêta-carbolin-6-yl}-2-(3-méthylphényl)acétamide ;
5-méthyl-N-{4-[2-(méthylamino)-2-oxoéthyl]-1-oxo-2,9-dihydro-1H-bêta-carbolin-6-yl}pyrazine-2-carboxamide ;
5-bromo-N-{4-[2-(méthylamino)-2-oxoéthyl]-1-oxo-2,9-dihydro-1H-bêta-carbolin-6-yl}-2-furamide ;
N-méthyl-2-(1-oxo-6-{[(pyridin-4-ylthio)acétyl]amino}-2,9-dihydro-1H-bêta-carbolin-4-yl)acétamide ;
N-benzyl-2-(6-nitro-1-oxo-2,9-dihydro-1H-bêta-carbolin-4-yl)acétamide ;
N-isopropyl-2-(6-nitro-1-oxo-2,9-dihydro-1H-bêta-carbolin-4-yl)acétamide ;
N-méthyl-2-(6-nitro-1-oxo-2,9-dihydro-1H-bêta-carbolin-4-yl)acétamide ; et

N-[4-(2-hydroxyéthyl)-1-oxo-2,3,4,9-tétrahydro-1H-bêta-carbolin-6-yl]-2,2-diméthylpropanamide.

**3.** Composé selon la revendication 1, dans lequel R est-$CONR^2R^3$ ; et $R^1$ est -$NHR^4$ ou -$NHCOR^4$.

**4.** Composé selon la revendication 1, dans lequel R est-$CONR^2R^3$, dans lequel $R^2$ et $R^3$ sont chacun indépendamment un atome d'hydrogène ou un groupe alkyle ; et $R^1$ est -$NHCOR^4$.

**5.** Composition pharmaceutique comprenant une quantité du composé selon la revendication 1, ou un sel ou un solvate pharmaceutiquement acceptable de celui-ci, et un véhicule pharmaceutiquement acceptable.

**6.** Composé selon la revendication 1 ou composition pharmaceutique selon la revendication 5, pour un usage thérapeutique.

**7.** Composé ou composition pharmaceutique selon la revendication 6, dans lequel ledit usage thérapeutique est pour le traitement d'un trouble ou d'une affection de la prolifération cellulaire chez un mammifère.

**8.** Composé ou composition pharmaceutique selon la revendication 7, dans lequel ledit trouble ou ladite affection est provoqué par ou associé à une activité modifiée des kinases Cdk2 ou Cdc7.

**9.** Composé ou composition pharmaceutique selon la revendication 7, dans lequel ledit trouble ou ladite condition est sélectionné dans le groupe constitué d'un carcinome, d'un carcinome à cellules squameuses, de tumeurs hématopoïétiques de la lignée myéloïde ou lymphoïde, de tumeurs d'origine mésenchymateuse, de tumeurs du système nerveux central et périphérique, d'un mélanome, d'un séminome, d'un tératocarcinome, d'un ostéosarcome, de xeroderma pigmentosum, d'un kératoxanthome, d'un cancer folliculaire de la tyroïde, et du sarcome de Kaposi.

**10.** Composé ou composition pharmaceutique selon la revendication 7, dans lequel ledit trouble ou ladite affection est sélectionné dans le groupe constitué de l'hyperplasie bénigne de la prostate, de l'adénomatose familiale, de la polypose, de la neuro-fibromatose, du psoriasis, de la prolifération des cellules des muscles lisses vasculaires associée à l'athérosclérose, de la fibrose pulmonaire, de l'arthrite, de la glomérulonéphrite, de la sténose post-chirurgicale et de la resténose.

**11.** Composé ou composition pharmaceutique selon la revendication 7, dans lequel ledit trouble ou ladite condition est sélectionné dans le groupe constitué d'un carcinome, d'un carcinome à cellules squameuses, de tumeurs hématopoïétiques de la lignée myéloïde ou lymphoïde, de tumeurs d'origine mésenchymateuse, de tumeurs du système nerveux central et périphérique, d'un mélanome, d'un séminome, d'un tératocarcinome, d'un ostéosarcome, de xeroderma pigmentosum, d'un kératoxanthome, d'un cancer folliculaire de la tyroïde, et du sarcome de Kaposi.

**12.** Composé ou composition pharmaceutique selon la revendication 7, dans lequel ledit trouble ou ladite condition est sélectionné dans le groupe constitué d'un carcinome à cellules squameuses, de la leucémie, de la leucémie lymphocytaire aiguë, de la leucémie lymphoblastique aiguë, d'un lymphome à cellules B, d'un lymphome à cellules T, du lymphome d'Hodgkin, d'un lymphome non hodgkinien, d'un lymphome à tricholeucocytes et du lymphome de Burkitt, de leucémies myéloïdes aiguës et chroniques, d'un syndrome myélodysplasique, de la leucémie promyélocytaire, d'un fibrosarcome, d'un rhabdomyosarcome, d'un astrocytome, d'un neuroblastome, d'un gliome, de schwannomes, d'un mélanome, d'un séminome, d'un tératocarcinome, d'un ostéosarcome, de xeroderma pigmentosum, d'un kératoxanthome, d'un cancer folliculaire de la tyroïde, du sarcome de Kaposi et d'un carcinome de la vessie, du sein, du côlon, du rein, du foie, du poumon, de l'oesophage, de la vésicule biliaire, de l'ovaire, du pancréas, de l'estomac, du col de l'utérus, de la thyroïde, de la prostate, ou la peau.

**13.** Produit ou kit comprenant un composé de formule (I) ou un sel pharmaceutiquement acceptable de celui-ci, selon la revendication 1, ou des compositions pharmaceutiques selon la revendication 5, et un ou plusieurs agents chimiothérapeutiques, sous la forme d'une préparation combinée pour une utilisation simultanée, séparée ou séquentielle dans une thérapie anticancéreuse.

**14.** Utilisation d'un composé de formule (I) ou d'un sel pharmaceutiquement acceptable de celui-ci, selon la revendication 1, dans la fabrication d'un médicament ayant une activité antitumorale.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 03022849 A
- WO 00002878 A
- WO 0212242 A
- WO 0069846 A
- WO 0198299 A
- WO 03014090 A
- WO 03028720 A
- WO 2003027114 A
- WO 2004058762 A
- US 20060276496 A


### Non-patent literature cited in the description


- *Current Opinion in Chemical Biology,* 1999, vol. 3, 459-465
- **MONTAGNOLI A. et al.** *EMBO Journal,* 2002, vol. 21 (12), 3171-3181
- **MONTAGNOLI A. et al.** *Cancer Research,* 01 October 2004, vol. 64, 7110-7116
- **WU JIANG-PING et al.** The discovery of carboline analogs as potent MAPKAP-K2 inhibitors. *BIOORGANIC & MEDICINAL CHEMISTRY LETTERS,* vol. 17 (16), ISSN 0960-894X, 4664-4669
- **T. HIGUCHI ; V. STELLA.** Pro-drugs as Novel Delivery Systems. A.C.S. Symposium Series, 1987, vol. 14
- Bioreversible Carriers in Drug Design. American Pharmaceutical Association and Pergamon Press, 1987
- **MAKLAKOV et al.** *Chem Heterocycl compd,* 2002, vol. 38 (5), 539-542
- **TOIS et al.** *Tetrahedron Lett.,* 2000, vol. 41 (14), 2443-2446